# EUROPEAN PATENT APPLICATION

(11) **EP 3 971 180 A1**
(43) Date of publication of application: **23.03.2022**
(21) Application number: 20802566.8
(22) Date of filing: 14.04.2020
(51) Int. Cl.: C07D 405/06, C07D 413/06, C07D 417/06, C07D 405/12, C07D 471/04, C07D 401/06, C07D 411/06, C07D 209/44, C07D 319/18, A61K 31/4035, A61K 31/4725, A61K 31/538, A61K 31/4025, A61K 31/4433, A61K 31/506

(54) **COMPOUNDS INHIBITING TDG ACTIVITY**

(30) Priority: 09.05.2019 CN 201910385851
(71) Applicant: Epitas Biosciences (Shanghai) Co., Ltd., Shanghai 201203 (CN)
(72) Inventor: ZHU, Weixing, Shanghai 201203 (CN); YANG, Shenglian, Shanghai 201203 (CN); XU, Guoliang, Shanghai 201203 (CN)
(74) Representative: Nederlandsch Octrooibureau
(86) International application number: PCT/CN2020/084755
(87) International publication number: WO 2020/224396

(57) **Abstract**

The present invention provides a class of compounds inhibiting TDG activity. Specifically, the present invention provides a compound having a novel structure as shown in formula I. The small molecule inhibitor of the present invention has an excellent inhibitory effect on TDG.

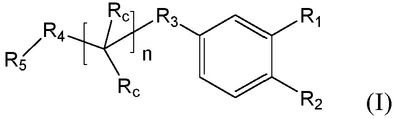

## Description

### TECHNICAL FIELD

The present invention relates to the field of medicinal chemistry, and in particular to a compound inhibiting TDG activity and preparation and use thereof.

### BACKGROUND

Human thymine DNA glycosylase (hTDG) belongs to the superfamily of uracil DNA glycosylase (UDG). Enzymes in this family use the base-flipping mechanism to locate damaged bases in double-stranded DNA (dsDNA), and mediate DNA repair by activating DNA base-excision repair (BER) pathways to replace the damaged bases. It is reported that hTDG recognizes mismatched pyrimidines uracil and thymine in G·U and G·T pairings, and then performs glycosyl bond cleavage to activate the BER pathway to repair these mismatched DNAs. Another important function of hTDG is to participate in the epigenetic regulation pathway by mediating the active demethylation of 5-methylcytosine (5mC). In eukaryotes, methylation and demethylation at the C5 position of cytosine play a crucial role in transcription regulation and genome reprogramming. Recent studies have shown that in mammals, members of the TET dioxygenase family can oxidize 5mC to 5-hydroxymethylcytosine (5hmC), and further oxidize to 5-formylcytosine (5fC) and 5caC. After hTDG recognizes 5caC and 5fC, it activates the BER pathway, excises oxidized cytosine and repairs the excision site with unoxidized cytosine (C), and finally completes the active demethylation process of 5mC. hTDG participates in transcriptional regulation and mouse embryonic development through this newly discovered active demethylation process. In addition, hTDG can also bind to a series of transcriptional regulators (such as CBP/p300, RXRa, TCF4, TTF-1, etc.) through protein-protein interactions, and participate in cell transcriptional regulation.

In addition to participating in mouse embryonic development, TDG expression levels increase in breast tumor epithelial tissue, osteosarcoma, and lymphocytic carcinoma in a variety of transgenic carcinogenic mouse models. Studies have shown that the expression of TDG protein is up-regulated in clinicopathological slices of colorectal cancer and gastric cancer, and the nucleoside polymorphism of the TDG gene is closely related to the susceptibility of a variety of tumors. Using gene interference technology to reduce the expression of TDG can inhibit the survival and growth of a variety of tumor cell lines such as lung cancer, colorectal cancer, melanoma, breast cancer, liver cancer, glioma, kidney cancer, pancreatic cancer, ovarian cancer, gastric cancer, neuroblastoma, etc.

However, there is still a lack of high-efficiency small molecule inhibitors targeting TDG.

In summary, there is an urgent need in the art to develop an efficient small molecule inhibitor targeting TDG.

### SUMMARY

A purpose of the present invention is to provide an efficient small molecule inhibitor targeting TDG.

In a first aspect of the present invention, provided is a compound represented by formula I, 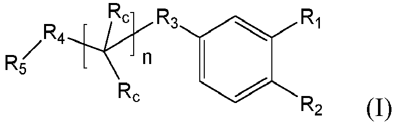 or a stereoisomer or a tautomer, or a pharmaceutically acceptable salt, or a hydrate, or a crystal form, or a solvate thereof,
wherein,
R₁ and R₂ are each independently selected from the group consisting of OH, SH, substituted or unsubstituted C1-6 alkoxy (-O-C1-6 alkyl), substituted or unsubstituted C1-6 alkylthio (-S-C1-6 alkyl); or R₁ and R₂ taken together with the carbon atom to which they are attached form a substituted or unsubstituted five- to seven-membered ring; and the five- to seven-membered ring is selected from the group consisting of 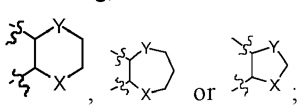 wherein, X and Y are each independently selected from: C(R_{c})₂ (preferably CH₂), O, S, NRₐ, C(=O), C(=S);
in R₁ and R₂, the term "substituted" means that a hydrogen in the group is replaced by one or more (preferably 1-3) substituents selected from the group consisting of halogen, C1-6 alkyl, halogenated C1-6 alkyl, cyano;
R₃ is selected from the group consisting of C(R_{c})₂, C=O, C=S, CR_{c}(OH), CR_{c}(SH);
n is 0, 1, 2, 3 or 4;
R₄ is a divalent group selected from the group consisting of substituted or unsubstituted C2-C6 alkenylene, substituted or unsubstituted C2-C6 alkynylene, NRₐ, substituted or unsubstituted ring G group; wherein, the ring G is selected from the group consisting of a 4- to 18-membered heterocyclic ring (preferably, 4-membered to 12-membered heterocyclic ring, more preferably 4-membered to 6-membered heterocyclic ring), a 5- to 18-membered heteroaromatic ring (preferably, 5-membered to 12-membered heteroaromatic ring), a C3-C14 aromatic ring (preferably, C6-C10 aromatic ring; more preferably, benzene ring), and a C3-C18 carbocyclic ring;
in R₄, the term "substituted" means that one or more hydrogens in the R₄ group are each independently replaced by R' and/or R"; with the proviso that when R₄ is a ring G group, the ring G can further optionally comprise a oxo and/or a thio;
R₅ is selected from the group consisting of H, nitro, halogen (preferably, F, Cl, Br), cyano, substituted or unsubstituted C1-C12 alkyl, substituted or unsubstituted C1-C12 alkoxy (C1-C12 alkyl-O-)(preferably, C1-C6 alkoxy), substituted or unsubstituted C1-C12 alkylcarbonyl (C1-C12 alkyl-C(O)-)(preferably, C1-C6 alkylcarbonyl), -(C(R_{c})₂)ₒ-OCOR_{b},-(C(R_{c})₂)ₒ-COOR_{b}, -(C(R_{c})₂)ₒ-NRₐ-COOR_{b}, -(C(R_{c})₂)ₒ-NRₐ-OCOR_{b}, N(Rₐ)₂, substituted or unsubstituted ring E group; wherein the ring E group is selected from the group consisting of 4- to 18-membered heterocyclic group (preferably, 4-membered to 12-membered heterocyclic group), 5- to 18-membered heteroaryl group (preferably, 5- to 12-membered heteroaryl), C6-C14 aryl (preferably, C6-C10 aryl; more preferably, phenyl), C3-C18 cycloalkyl; in R₅, the term "substituted" means that one or more hydrogens in the R₅ group are replaced by R' and/or R"; with the proviso that when R₅ is a ring E group, the ring E group can further optionally comprise a oxo and/or a thio;
o = 0, 1 or 2;
R' is each independently selected from the group consisting of hydroxyl, thiol (-SH), nitro, halogen (preferably, F, Cl, Br), cyano, substituted or unsubstituted C1-C10 alkyl (preferably, C1-C6 alkyl, more preferably, C1-C4 alkyl), N(Rₐ)₂, substituted or unsubstituted C1-C10 alkoxy (preferably, C1-C6 alkoxy, more preferably, C1-C4 alkoxy), substituted or unsubstituted C1-C10 alkylthio (preferably, C1-C6 alkylthio, more preferably C1-C4 alkylthio), substituted or unsubstituted C1-C10 alkylcarbonyl (preferably, C1-C6 alkylcarbonyl, more preferably , C1-C4 alkylcarbonyl), -COOR_{b}, -OCOR_{b}, substituted or unsubstituted 4- to 12-membered heterocyclic group, substituted or unsubstituted 5- to 12-membered heteroaryl, substituted or unsubstituted C6-C14 aryl, substituted or unsubstituted C3-C15 carbocyclic group;
R" is each independently selected from the group consisting of H, hydroxyl, thiol, C1-C6 alkyl, C1-C6 haloalkyl;
Rₐ, R_{b} and R_{c} are each independently selected from the group consisting of H, C1-6 alkyl, C1-C6 haloalkyl;
unless otherwise specified, the term "substituted" means that one or more (preferably 1-3) hydrogens in the group are replaced by a substituent selected from the group consisting of cyano, hydroxy, halogen (F, Cl, Br, I), C1-6 alkyl, and C1-6 haloalkyl.

In another preferred embodiment, R' is each independently selected from the group consisting of nitro, halogen (preferably, F, Cl, Br), cyano, C1-C6 alkyl, C1-C6 haloalkyl, N(Rₐ)₂ (preferably, NH₂), C1-C6 alkoxy, halogenated C1-C6 alkoxy, C1-C6 alkylthio, halogenated C1-C6 alkylthio, C1-C6 alkylcarbonyl, halogenated C1-C6 alkylcarbonyl.

In another preferred embodiment, R' is each independently selected from the group consisting of nitro, NH₂, Br, Cl, C1-C4 alkoxy, C1-C4 alkyl, C1-C4 haloalkyl, cyano.

In another preferred embodiment, R4 is selected from the group consisting of C2-C6 alkenylene, NRₐ, substituted or unsubstituted ring G.

In another preferred embodiment, the ring G is a heterocyclic ring or heteroaromatic ring containing at least one N heteroatom; preferably, the N heteroatom in ring G is connected with

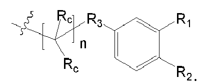

In another preferred embodiment, 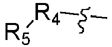 is R₅-NRₐ-, 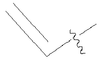 or 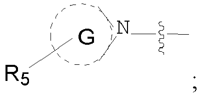 wherein, 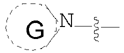 represents a substituted or unsubstituted ring G group.

In another preferred embodiment, the ring G group is selected from the group consisting of:

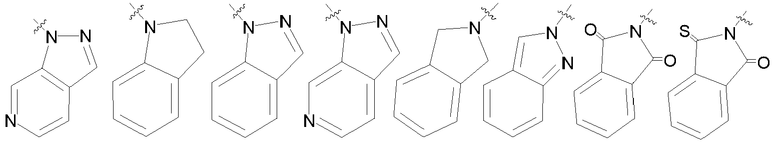

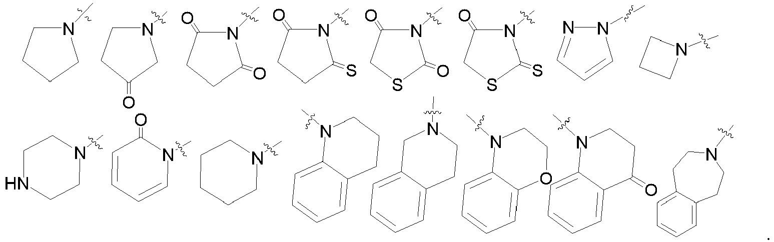

In another preferred embodiment, 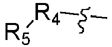 is a group selected from the following group that is unsubstituted or substituted with 1 to 3 R' and/or R": R₅-NRₐ-,

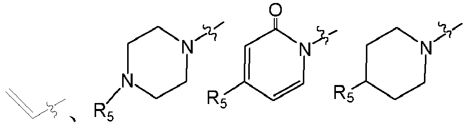

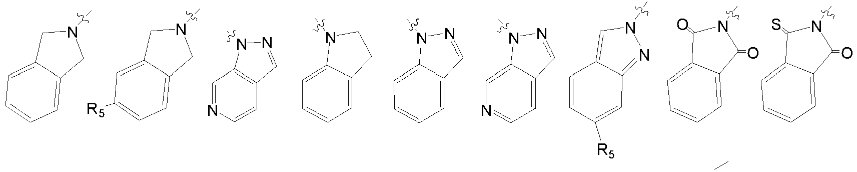

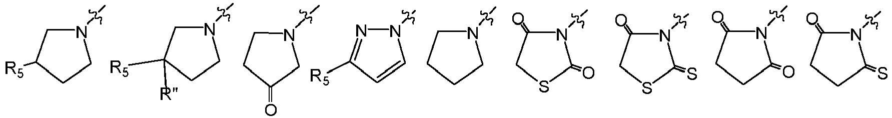

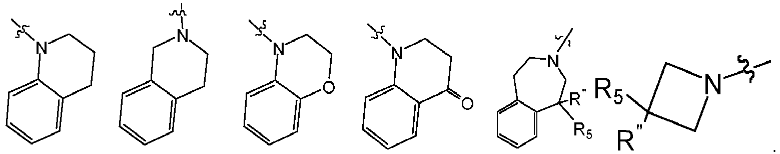

In another preferred embodiment, R₅ is selected from the group consisting of H, nitro, halogen (preferably, F, Cl, Br), cyano, C1-C12 alkyloxy (preferably, C1-C6 alkyloxy), C1-C12 alkylcarbonyl (preferably, C1-C6 alkylcarbonyl), C1-C12 alkyl ester group (preferably, C1-C6 alkyl ester group), -(C(R_{c})₂)ₒ-NRₐ-COOR_{b}, NRₐ, unsubstituted or 1-3 R' substituted ring E group.

In another preferred embodiment, the ring E group is selected from: C6-C14 aryl (preferably, phenyl), 5-membered to 10-membered heteroaryl (preferably, a 5- to 10-membered heteroaromatic ring containing 1-3 nitrogen heteroatoms).

In another preferred embodiment, the ring E group is selected from:

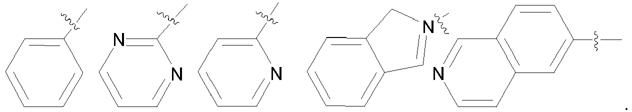

In another preferred embodiment, the ring E group is selected from:

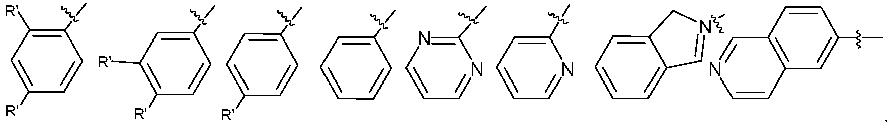

In another preferred embodiment, R₃ is absent or C=O.

In another preferred embodiment, the five- to seven-membered ring is 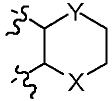 or

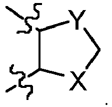

In another preferred embodiment, at least one of X and Y is O or S.

In another preferred embodiment, X is O, and Y is selected from: O, C=O.

In another preferred embodiment, the compound is represented by formula II, 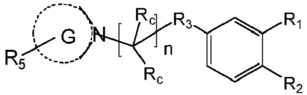 wherein, R₁, R₂, R₃, R₅, R_{c}, ring G and n are as defined above.

In another preferred embodiment, the compound is represented by formula III, 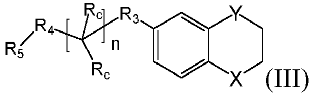 wherein, R₃, R₄, R₅, R_{c}, Y, X and n are as defined above.

In another preferred embodiment, the compound is represented by formula IV, 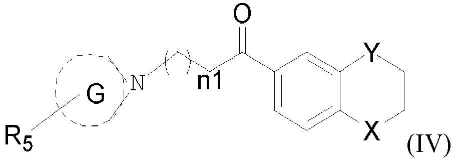 wherein, n1=0, 1 or 2; ring G, R₅, Y and X are defined as defined above.

In another preferred embodiment, the compound is represented by formula IV-A, formula IV-B, formula IV-C, or formula IV-D, 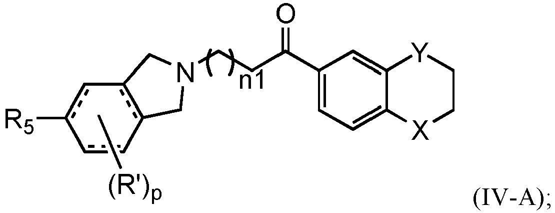 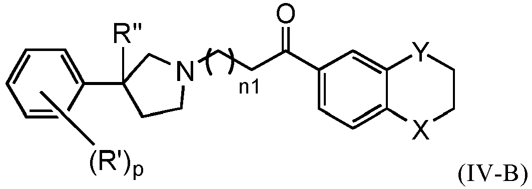 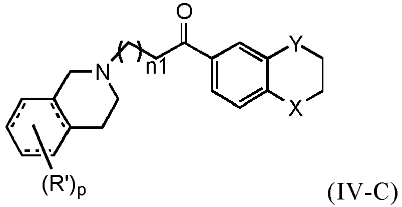 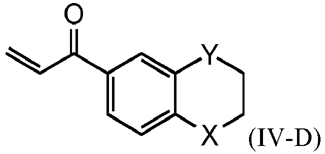 wherein, p=0, 1 or 2; n1=0, 1 or 2;
R₅, R', R", Y and X are as defined in claim 1.

In another preferred embodiment, the compound is represented by formula V-A or formula V-B, 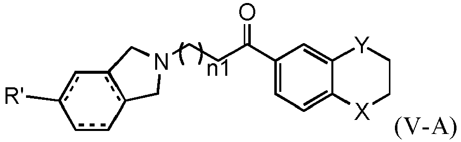 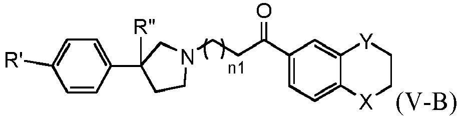 wherein, n1, R', R", Y and X are as defined above.

In another preferred embodiment, the compound is selected from compounds 1-54 in Table A.

The second aspect of the present invention provides a method for preparing the compound according to the first aspect, the method comprising: 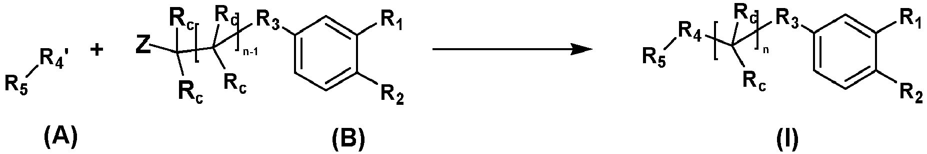 reacting a compound of formula A with a compound of formula B to obtain a compound of formula I;
wherein, Z is a leaving group;
n is 1, 2 or 3;
R₅-R₄' is selected from the group consisting of: 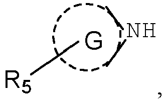 R₅-N(Rₐ)H;
R₁, R₂, R₃, R₅ and R_{c} are are as defined in the first aspect.

In another preferred embodiment, Z is selected from the group consisting of Cl, Br, I.

The third aspect of the present invention provides a pharmaceutical composition comprising (i) the compound as described in the first aspect and (ii) a pharmaceutically acceptable carrier.

The fourth aspect of the present invention provides use of the compound as described in the first aspect, or a salt, or an isomer thereof, or a pharmaceutical composition as described in the third aspect (i) for the preparation of a TDG inhibitor, or (ii) for the preparation of a medicament for treating and/or preventing a disease related to TDG overexpression.

In another preferred embodiment, the disease related to TDG overexpression is a tumor.

In another preferred embodiment, the tumor is selected from the group consisting of lung cancer, colorectal cancer, melanoma, breast cancer, liver cancer, glioma, kidney cancer, pancreatic cancer, ovarian cancer, gastric cancer, neuroblastoma, or a combination thereof.

The fifth aspect of the present invention provides a method for inhibiting TDG activity, the method comprising:
contacting a subject with an effective amount of the compound as described in the first aspect, or a stereoisomer or a tautomer, or a pharmaceutically acceptable salt, or a hydrate, or a crystal form, or a solvate thereof, or the pharmaceutical composition as described in the third aspect, thereby inhibiting TDG activity.

In another preferred embodiment, the method is non-therapeutic and non-diagnostic in vitro.

The sixth aspect of the present invention provides a method for treating and/or preventing a disease related to TDG overexpression, the method comprising:
administering to a subject an effective amount of the compound as described in the first aspect, or a salt, or an isomer thereof, or the pharmaceutical composition as described in the third aspect.

In another preferred embodiment, the subject is a mammal; preferably, a human.

It should be understood that within the scope of the present invention, the above-mentioned technical features of the present invention and the technical features specifically described in the following (such as in the examples) can be combined with each other to form a new or preferred technical solution, which will not be repeatedly described herein due to space limitation.

### DETAILED DESCRIPTION OF EMBODIMENTS

The inventors have gone through extensive and in-depth research. It was discovered that compounds with the novel structure as shown in formula I, especially a series of benzodioxane compounds as shown in formula I, have excellent inhibitory activity on TDG. Based on this, the present invention has been completed.

### Definitions

As used herein, the term "ring G group" refers to a (divalent) group formed by ring G losing two hydrogens on the ring. Similarly, "ring E group" refers to a group formed by ring E losing one hydrogen on the ring.

Unless otherwise specified, the term "alkyl" by itself or as part of another substituent refers to a linear or branched chain hydrocarbon group having the specified number of carbon atoms (i.e., C1-C8 represents 1-8 carbons). In a preferred example, alkyl generally refers to C1-C6 alkyl, preferably, C1-C4 alkyl. Examples of alkyl groups include, but are not limited to: methyl, ethyl, n-propyl, isopropyl, n-butyl, t-butyl, isobutyl, sec-butyl, n-pentyl, n-hexyl, n-heptyl, n-octyl and so on.

Unless otherwise specified, the term "alkenyl" or "alkenylene" refers to an unsaturated alkyl or alkylene group having one or more double bonds. Similarly, the term "alkynyl" or "alkynylene" refers to an unsaturated alkyl or alkylene group having one or more triple bonds. Examples of such unsaturated alkyl groups include vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl and higher homologs and isomers.

Unless otherwise specified, the term "cycloalkyl" or "carbocyclic ring" refers to a hydrocarbon ring that has the specified number of ring atoms (e.g., C3-C18 cycloalkyl or C3-C18 carbocyclic ring) and is fully saturated or unsaturated (for example, there is no more than one double bond between the ring vertexs). For example, cyclopropyl (alkyl), cyclobutyl (alkyl), cyclopentyl (alkyl) and the like. "Cycloalkyl" or "carbocyclic ring" also includes bicyclic and polycyclic hydrocarbon rings, such as bicyclo[2.2.1]heptane, bicyclo[2.2.2]octane and the like.

Unless otherwise specified, the term "heterocyclyl" or "heterocycle" refers to a saturated or unsaturated cycloalkyl group containing one to five heteroatoms selected from N, O, and S, wherein the nitrogen and sulfur atoms are optionally oxidized, and the nitrogen atom is optionally quaternized. The heterocyclic group can be a monocyclic, bicyclic or polycyclic ring system. Non-limiting examples of heterocyclic groups include pyrrolidine, imidazolidine, pyrazolidine, butyrolactam, valerolactam, imidazolidinone, hydantoin, dioxolane, phthalimide, piperidine, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, quinuclidine, etc. The heterocycloalkyl group can be attached to the rest of the molecule via a ring carbon or a heteroatom. For terms such as cycloalkyl and heterocyclylalkyl, it is meant that the cycloalkyl or heterocycloalkyl is attached to the rest of the molecule through an alkyl or alkylene linker.

Unless otherwise specified, the term "alkylene" by itself or as part of another substituent refers to a divalent group derived from an alkane, such as -CH₂CH₂CH₂CH₂-. An alkyl group (or alkylene group) generally has 1 to 24 carbon atoms, with those having 10 or less carbon atoms being preferred in the present invention. "Lower alkyl" or "lower alkylene" is a shorter chain alkyl or alkylene group, usually having 4 or fewer carbon atoms. Similarly, "alkenylene" or "alkynylene" refers to an unsaturated form of "alkylene" having a double bond or a triple bond, respectively.

Similarly, unless otherwise specified, the terms "heteroalkenyl" and "heteroalkynyl" by themselves or in combination with another term refer to alkenyl or alkynyl, respectively, which contain the specified number of carbons and 1 to 3 heteroatoms selected from O, N, Si and S, respectively, and wherein the nitrogen and sulfur atoms are optionally oxidized, and the nitrogen heteroatom may optionally be quaternized. The heteroatoms O, N and S can be located in any internal position of the heteroalkyl group.

Unless otherwise specified, the terms "alkoxy" or "alkyloxy", "alkamino" or "alkylamino" and "alkthio" or "alkylthio" (or thioalkoxy) are used in their conventional sense and refer to alkyl groups attached to the rest of the molecule via an oxygen atom, an amino group, or a sulfur atom, respectively. In addition, for a dialkylamino group, the alkyl groups can be the same or different, and can also be combined with the nitrogen atoms connected to respective alkyl groups to form a 3-7 membered ring. Therefore, the group represented by - NR^{a}R^{b} includes piperidinyl, pyrrolidinyl, morpholinyl, azetidinyl and the like.

Unless otherwise specified, the term "halo" or "halogen" by itself or as part of another substituent refers to a fluorine, chlorine, bromine, or iodine atom. In addition, terms such as "haloalkyl" are meant to include monohaloalkyl or polyhaloalkyl. For example, the term "C₁₋₄ haloalkyl" is meant to include trifluoromethyl, 2,2,2-trifluoroethyl, 4-chlorobutyl, 3-bromopropyl and the like.

Unless otherwise specified, the term "aryl" or "aromatic ring" refers to a polyunsaturated (usually aromatic) hydrocarbon group, which can be a single ring or multiple rings (up to three rings) fused or covalently linked together. The term "heteroaryl" or "heteroaromatic ring" refers to an aryl group (or ring) containing 1 to 5 heteroatoms selected from N, O, and S, wherein the nitrogen and sulfur atoms are optionally oxidized, and the nitrogen atom is optionally quaternized. A heteroaryl group can be attached to the rest of the molecule through a heteroatom. Non-limiting examples of aryl groups include phenyl, naphthyl, and biphenyl, while non-limiting examples of heteroaryl groups include pyridyl, pyridazinyl, pyrazinyl, pyrimidinyl, triazinyl, quinolinyl, quinoxalinyl, quinazolinyl, cinnoline, phthalazinyl, benzotriazinyl, purinyl, benzimidazolyl, benzopyrazolyl, benzotriazolyl, benzoisoxazolyl, isobenzofuryl, isoindolyl, indolizinyl, benzotriazinyl, thienopyridyl, thienopyrimidinyl, pyrazolopyrimidinyl, imidazopyridine, benzothiazolyl, benzofuranyl, benzothienyl, indolyl, quinolinyl, isoquinolinyl, isothiazolyl, pyrazolyl, indazolyl, pterridinyl, imidazolyl, triazolyl, tetrazolyl, oxazolyl, isoxazolyl, thiadiazolyl, pyrrolyl, thiazolyl, furyl, thienyl and the like. The respective substituents of the above aryl and heteroaryl ring systems are selected from the group of acceptable substituents described below.

For the sake of brevity, when the term "aryl" is used in combination with other terms (e.g., aryloxy, arylthio, aralkyl), it includes aryl and heteroaryl rings as defined above. Therefore, the term "aralkyl" is meant to include those groups in which the aryl group is attached to an alkyl group attached to the rest of the molecule (e.g., benzyl, phenethyl, pyridylmethyl, etc.).

In some embodiments, the aforementioned terms (such as "alkyl", "aryl" and "heteroaryl") will include both substituted and unsubstituted forms of the specified groups. The preferred substituents for each type of group are provided below. For the sake of brevity, the terms aryl and heteroaryl will refer to the substituted or unsubstituted forms as provided below, while the term "alkyl" and related aliphatic groups refer to the unsubstituted form, unless it is indicated as being substituted.

The substituents for the alkyl group (including those commonly referred to as alkylene, alkenyl, alkynyl and cycloalkyl) may be various groups selected from the following group:-halogen, -OR', -NR'R", -SR', -SiR'R"R"', OC(O)R', -C(O)R', -CO₂R', -CONR'R",-OC(O)NR'R", -NR"C(O)R', -NR'-C(O)NR"R"', -NR"C(O)₂R', -NH-C(NH₂)=NH,-NR'C(NH₂)=NH, -NH-C(NH₂)=NR', -S(O)R', -S(O)₂R', -S(O)₂NR'R", NR'S(O)₂R", -CN and-NO₂, with the number ranging from zero to (2m'+1), where m' is the total number of carbon atoms in this group. R', R" and R"' each independently represent hydrogen, unsubstituted C₁₋₈ alkyl, unsubstituted heteroalkyl, unsubstituted aryl, aryl substituted by 1-3 halogens, unsubstituted C₁₋₈ alkyl, C₁₋₈ alkoxy or C₁₋₈ thioalkoxy, or unsubstituted aryl-C₁₋₄ alkyl. When R' and R" are attached to the same nitrogen atom, they can combine with the nitrogen atom to form 3-, 4-, 5-, 6- or 7-membered ring. For example, -NR'R" means to include 1-pyrrolidinyl and 4-morpholinyl. The term "acyl", used alone or as part of another group, refers to one in which the two substituents on the carbon closest to the point of attachment of the group are substituted with the substituent =O (for example, -C(O)CH₃, -C(O)CH₂CH₂OR', etc.).

As used herein, the term "heteroatom" is intended to include oxygen (O), nitrogen (N), sulfur (S), and silicon (Si).

For the compounds provided herein, the bond from the substituent (usually the R group) to the center of the ring (e.g., ring G, ring E, etc.) will be understood to mean the bond that provides the connection at any available vertex of the ring (or a group derived from the ring).

Unless otherwise specified, in the present invention, all compounds appearing are intended to include all possible optical isomers, such as a single chiral compound, or a mixture of various different chiral compounds (i.e., racemates). In all the compounds of the present invention, each chiral carbon atom may optionally be in R configuration or S configuration, or a mixture of R configuration and S configuration.

Certain compounds of the present invention possess asymmetric carbon atoms (optical centers) or double bonds; racemates, diastereomers, geometric isomers, regioisomers and individual isomers (e.g., separated enantiomers) should be included in the scope of the present invention. When the compounds provided herein have a defined stereochemistry (represented by R or S, or indicated by a dashed or wedge-shaped bond), those skilled in the art will understand that those compounds are substantially free of other isomers (e.g., at least 80%, 90%, 95%, 98%, 99% and up to 100% free of other isomers).

In this description, unless otherwise specified, the term "substituted" means that one or more hydrogen atoms on the group are replaced by a substituent selected from the following group: cyano, hydroxyl, halogen (F, Cl, Br, I), C1-6 alkyl, C1-6 haloalkyl.

As used herein, "1 to 3" or "1-3" refers to 1, 2, or 3.

As used herein, the term "comprise", "comprises" or "comprising" means that various ingredients can be used together in the mixture or composition of the present invention. Therefore, the terms "substantially consisted of" and "consisted of" are included in the term "comprising".

As used herein, the term "pharmaceutically acceptable" ingredient refers to a substance that is suitable for use in humans and/or animals without excessive adverse side effects (such as toxicity, irritation, and allergic reactions), that is, a substance that has a reasonable benefit/risk ratio.

As used herein, the term "therapeutically effective dosage" or "effective amount" refers to any amount of a drug as described below, which when used alone or in combination with another therapeutic agent, can promote the regression of the disease. Regression is manifested as a decrease in the severity of disease symptoms, an increase in the frequency and duration of the asymptomatic period, or prevention of disorder or disability caused by the disease. The "therapeutically effective dosage" or "effective amount" of the drug or medicament of the present invention also includes the "prophylactic effective dosage". The "prophylactic effective dosage" is any amount of the drug as described below, and when this amount of the drug is administered alone or in combination with another therapeutic agents is administered to a subject who is at risk of developing a disease or suffering from a recurrence of the disease, the occurrence or recurrence of the disease can be suppressed.

### Compounds of the Invention

The present invention provides compounds for efficiently inhibiting TDG.

As used herein, the term "compound of the present invention" or "active compound" refers to a compound having the structure of formula I, 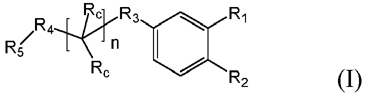 or a pharmaceutically acceptable salt, a prodrug, an optical isomer, a racemate, a solvate, a multimer thereof,
wherein, each group is defined as described in the first aspect.

In a specific embodiment, the compound of the present invention is a compound in Table A (i.e., compound 1-compound 54).

As used herein, the term "pharmaceutically acceptable salt" refers to a salt formed by a compound of the present invention and an acid or base suitable for use as a medicine. Pharmaceutically acceptable salts include inorganic salts and organic salts. A preferred class of salts is the salts formed by the compound of the present invention and acids. Acids suitable for salt formation include, but are not limited to: inorganic acids such as hydrochloric acid, hydrobromic acid, hydrofluoric acid, sulfuric acid, nitric acid, phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, and benzenesulfonic acid; and acidic amino acids such as aspartic acid and glutamic acid.

In addition to the salt form, the present invention also provides the compound in the form of a prodrug. Prodrugs of the compounds described herein are those compounds that readily undergo chemical changes under physiological conditions to provide the compounds of the invention. In addition, prodrugs can be converted into compounds of the present invention by chemical or biochemical methods in an ex vivo environment. For example, when placed in a transdermal patch reservoir containing a suitable enzyme or chemical reagent, the prodrug can be slowly converted to the compound of the invention.

Certain compounds of the present invention can exist in unsolvated as well as solvated forms, including hydrated forms. The solvated form is generally equivalent to the unsolvated form and should be included in the scope of the present invention. Certain compounds of the present invention may exist in a polymorphic or amorphous form. Generally, as far as the application considered in the present invention is concerned, all physical forms are equivalent and should be included in the scope of the present invention.

A compound of the present invention may also contain an unnatural proportion of atomic isotopes at one or more of the isotopic atoms constituting the compound. An unnatural proportion of a certain isotope can be defined as from the naturally found amount of the atom concerned to 100% of that atom. For example, the compound may be incorporated with radioactive isotopes, such as tritium (³H), iodine-125 (¹²⁵I), or carbon-14 (¹⁴C), or non-radioactive isotopes, such as deuterium (²H) or carbon-13 (¹³C). In addition to those uses described in the present application, such isotopic variants may provide additional uses. For example, isotopic variants of the compounds of the invention may have additional uses, including, but not limited to, as diagnostic and/or imaging reagents, or as cytotoxic/radiotoxic therapeutic agents. In addition, isotopic variants of the compounds of the present invention may have altered pharmacokinetic and pharmacodynamic characteristics, thereby helping to increase safety, tolerability or efficacy during treatment. Regardless of whether it is radioactive or not, all isotopic variants of the compounds of the present invention should be included within the scope of the present invention.

The "pharmaceutical composition" of the present invention can be made into tablets, capsules, powders, granules, lozenges, suppositories, liquid preparation forms such as oral liquids or sterile parenteral suspensions, as well as injectable forms such as large or small volume injections, freeze-dried powders, etc. The drugs or medicaments in the above dosage forms can be prepared according to conventional methods in the field of pharmacy.

The "pharmaceutically acceptable carrier" described in the present invention includes conventional diluents, fillers, binders, wetting agents, absorption promoters, surfactants, adsorption carriers, lubricants, and the like in the pharmaceutical field.

As is obvious to those skilled in the art, the effective in vivo dosage and the specific mode of administration will vary according to the type, weight and age of the mammal being treated, the specific compound used and the specific purpose of using these compounds. Those skilled in the art can determine the effective dose level (i.e., the dose level necessary to achieve the desired effect) according to conventional pharmacological methods. Generally, the human clinical application of the product starts from a lower dosage level, and then the dosage level is continuely increased until the desired effect is achieved. Alternatively, useful doses and routes of administration of the compositions identified by the present method can be established by existing pharmacological methods using acceptable in vitro studies.

The administration mode of the compound of the present invention, its isomer, its pharmaceutically acceptable salt, or the pharmaceutical composition containing the compound is not particularly limited. Representative administration methods include (but are not limited to): oral, intratumoral, rectal, parenteral (intravenous, intramuscular, or subcutaneous) and the like. The compounds of the present invention can be administered alone or in combination with other pharmaceutically acceptable compounds. The compounds of the present invention can refer to the administration or administration methods of other small molecule inhibitors. For example, optional administration methods include (but are not limited to): oral, transdermal, intravenous, intramuscular, local, and the like.

### Methods of Preparation

The compounds of the present invention can be prepared by general methods in the art and/or by referring to the methods in the specific examples.

In a specific embodiment, the present invention provides a method for preparing the compound of formula I, comprising: 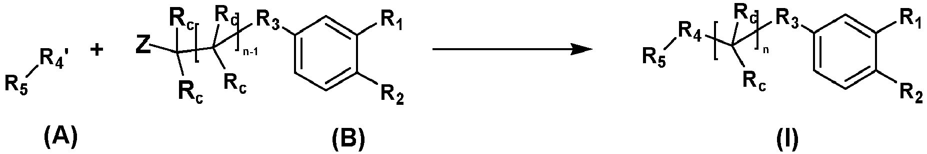 reacting a compound of formula A with a compound of formula B to obtain a compound of formula I;
wherein, Z is a leaving group, R4' contains an amine group that can undergo nucleophilic substitution reaction, n is 1, 2 or 3, and the other groups in the formula are as defined above.

Preferably, R₅-R₄' is selected from the group consisting of: 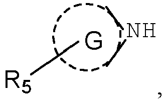 R₅-N(Rₐ)H;

Preferably, Z includes: Cl, Br, I.

### Pharmaceutical Compositions and Methods of Administration

Because the compound of the present invention has excellent TDG inhibitory activity, the compound of the present invention and its various crystal forms, pharmaceutically acceptable inorganic or organic salts, hydrates, solvates, polymers, and the pharmaceutical composition containing the compound of the present invention as the main active ingredients can be used to treat, prevent and alleviate diseases mediated by mutant TDG.

Preferably, the compounds of the present invention can be used to treat diseases associated with tumor cell lines. Representative diseases associated with tumor cell lines include (but are not limited to): lung cancer, colorectal cancer, melanoma, breast cancer, liver cancer, glioma, kidney cancer, pancreatic cancer, ovarian cancer, gastric cancer, neuroblastoma , or a combination thereof.

The pharmaceutical composition of the present invention contains a safe and effective amount of the compound of the present invention or a pharmacologically acceptable salt thereof and a pharmacologically acceptable excipient or carrier. The "safe and effective amount" refers to: the amount of the compound is sufficient to significantly improve the condition without causing serious side effects. Generally, the pharmaceutical composition contains 1-2000 mg of the compound of the present invention per dose, and more preferably, contains 10-500 mg of the compound of the present invention per dose. Preferably, the "one dose" is a capsule or tablet.

"A pharmaceutically acceptable carrier" refers to: one or more compatible solid or liquid fillers or gel substances, which are suitable for human use, and must have sufficient purity and sufficiently low toxicity. "Compatibility" here means that the components in the composition can be blended with the compound of the present invention and with each other without significantly reducing the efficacy of the compound. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween^{®}), wetting agents (such as sodium lauryl sulfate), coloring agents, flavoring agents, stabilizers, antioxidants, preservatives, pyrogen-free water, etc.

The method of administration of the compound or the pharmaceutical composition of the present invention is not particularly limited, and representative administration methods include (but are not limited to): oral, intratumoral, rectal, parenteral (intravenous, intramuscular, or subcutaneous), and local administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with the following ingredients: (a) fillers or compatibilizers, for example, starch, lactose, sucrose, glucose, mannitol and silicic acid; (b) binders such as hydroxymethyl cellulose, alginate, gelatin, polyvinylpyrrolidone, sucrose and gum arabic; (c) humectants, such as glycerin; (d) disintegrants, such as agar, calcium carbonate, potato starch or tapioca starch, alginic acid, certain complex silicates, and sodium carbonate; (e) sustained-releasing agents, such as paraffin; (f) absorption accelerators, such as quaternary amine compounds; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium lauryl sulfate, or mixtures thereof. In capsules, tablets and pills, the dosage form may also contain buffering agents.

Solid dosage forms such as tablets, sugar pills, capsules, pills and granules can be prepared with coatings and shell materials, such as enteric coatings and other materials known in the art. They may contain opacifying agents, and the active compound or compound in the composition can be released in a certain part of the digestive tract in a delayed manner. Examples of embedding components that can be used are polymeric substances and waxes. If necessary, the active compound can also be formed into microcapsules with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition may also contain adjuvants such as wetting agents, emulsifying agents and suspending agents, sweetening agents, flavoring agents and perfumes.

In addition to the active compound, the suspension may contain suspending agents, for example, ethoxylated isostearyl alcohol, polyoxyethylene sorbitol and sorbitan esters, microcrystalline cellulose, aluminum methoxide and agar, or mixtures of these substances, and the like.

The composition for parenteral injection may contain physiologically acceptable sterile aqueous or non-aqueous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

The dosage forms of the compound of the present invention for local administration include ointments, powders, patches, sprays and inhalants. The active ingredient is mixed under sterile conditions with a physiologically acceptable carrier and any preservatives, buffers, or propellants that may be required if necessary.

The compounds of the present invention can be administered alone or in combination with other pharmaceutically acceptable compounds.

When using the pharmaceutical composition, a safe and effective amount of the compound of the present invention is administered to a mammal (such as a human) in need of treatment. The administered dose is the effective dosage considered pharmaceutically. For a person with a body weight of 60 kg, the daily dosage is usually 1 to 2000 mg, preferably 20 to 500 mg. Of course, the specific dosage should also consider factors such as the route of administration, the patient's health status, etc., which are within the range of a skilled physician.

**The main advantages of the present invention include:**
(a) The compounds of the present invention have excellent inhibitory effect on TDG;
(b) The compounds of the present invention can effectively inhibit tumors and/or cancer cells.

The present invention will be further explained below in conjunction with specific examples. It should be understood that these examples are only used to illustrate the present invention and not to limit the scope of the present invention. The experimental methods wherein specific conditions are not indicated in the following examples usually follow the conventional conditions or the conditions recommended by the manufacturer. Unless otherwise specified, percentages and parts are percentages and parts by weight.

In this description, unless otherwise specified, the abbreviations refer to the conventional meanings known to those skilled in the art. For example, THF refers to tetrahydrofuran; DMF refers to N,N-dimethylformamide; DCM refers to dichloromethane; NBS refers to N-bromosuccinimide; AIBN refers to azobisisobutyronitrile; EA refers to ethyl acetate; PE refers to petroleum ether; DMAP refers to 4-dimethylaminopyridine; Et₃N refers to triethylamine; CAN refers to acetonitrile; HATU refers to 2-(7-azabenzotriazole) - N,N,N',N'-tetramethylurea hexafluorophosphate.

### Example 1

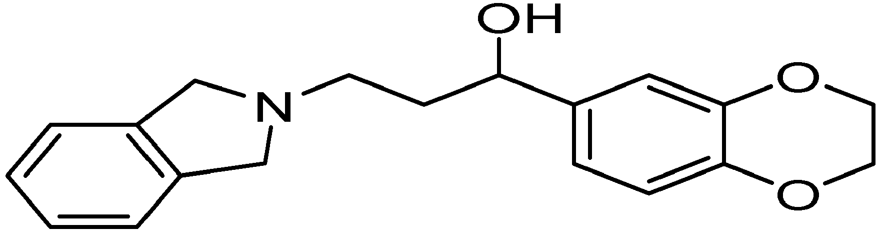

### Route 1. Synthesis of compounds 1-8.

### 1. General preparation method of compounds 1-8.

### 1.1.1 Synthesis of 3-bromo-1-(2,3-dihydrobenzo[b][1,4]dioxin-6-yl)propan-1-one (1i)

2,3-dihydrobenzo[b][1,4]dioxin 2 g (14.7 mmol) and 5 ml of DCM were mixed in a 50 ml vial. The mixture was cooled to 10°C. 3.15 g (18.4 mmol) of 3-bromopropyl chloride was added. Then 3.15 g of AlCl₃ was added in batches. The mixture was stirred at room temperature for 2 hours. After cooling to 10°C with an ice bath, the reaction stopped. Four milliliters of hydrochloric acid was slowly added dropwise. Then 20 ml of cold water was added to the reaction mixture and it was stirred for 10 min, the organic matter was collected, and the aqueous layer was extracted with DCM 20 ml×2. The composite organics were washed with saturated NaHCO₃ 5 ml×3, brine 10 ml, and concentrated in vacuo to obtain the crude product 1i.

### 1.1.2. Synthesis of 1,2-bisbromomethylbenzene (1b)

To a solution of 1,2-dimethylbenzene (14 mmol), was added NBS (35 mmol) AIBN (3.5 mmol) in 15 ml CCl₄, and the mixture was stirred at 70°C for 2 hours. The organic solvent was concentrated under vacuum to obtain the crude product, which was purified by flash column chromatography on an 80 g silica gel column with EA/PE gradient elution (0-10%) to obtain 1b.

### 1.1.3. Synthesis of 2-(toluene-4-sulfonyl)-2,3-dihydro-1H-isoindole (1c)

To a solution of NaH (0.5 g, 20.1 mmol) in 15 ml of DMF, was added Tos-NH₂ (1.1 g, 6.7 mmol) in 2.5 ml of DMF, and the mixture was stirred for 30 min at 60°C. After 30 min, 1b (6.7 mmol) in 2.5 ml of DMF was added dropwise to the mixed reaction. Then it was stirred at 60°C for 1 hour. 10 ml of ice water was added into the ice bath. After the mixture was allowed to stand still to separate into layers, the aqueous layer was extracted with EA 20ml×3. Then the organic layers were combined and washed with 10 ml×2 of NaHCO₃ saturated solution and 10 ml of brine. The solvent was removed in vacuo to obtain crude product 1c, which was purified by flash column chromatography on a 40 g silica gel column with gradient elution of EA/PE (80-100%).

### 1.1.4. Synthesis of 5-substituted-2,3-dihydro-1H-isoindole (1ii)

A mixture of 1c (2.60 mmol) in 12 ml of HBr/THF was stirred at room temperature or heated overnight. The solvent was concentrated under vacuum to obtain the crude product 1ii.

### 1.1. Synthesis of 3-(5-substituted-1,3-dihydro-isoindol-2-yl)-1-(2,3-dihydro-benzo[1,4]dioxin-6-yl)-propan-1-one (1)

1ii (2.6 mmol), Et₃N (788 mg, 7.8 mmol), and DMAP (61 mg, 0.5 mmol) were added to a solution of 20 ml THF, and 1i (589 mg, 2.6 mmol) was added. Then it was stirred at room temperature for 2 hours. 10ml of water was added to the reaction mixture, the mixture was stirred for 10min, allowed to stand still and separate into layers, and then the aqueous layer was extracted with EA 10ml×3. Then the organic layers were combined, washed with 5 ml×2 of NaHCO₃ and 5 ml of brine. The solvent was removed in vacuo to obtain a crude product. Purification by flash column chromatography on a 40 g silica gel column with gradient elution of EA/PE (80-100%) was used to obtain 400 mg of white solid (50% yield). LC-MS: RT = 1.29 min; Purity 97.3%; ESI m/z [M+1]⁺ =311.14. ¹HNMR (500 MHz, CDCl₃): δ 7.79-7.68 (m, 1H), 7.50-7.45 (m, 1H) ,7.27-7.21 (m, 4H), 7.14-7.03(m, 1H), 4.50-4.43 (m, 2H), 4.15-4.10 (m, 4H), 3.33 (t, *J=* 4.5Hz, 4H), 3.19-3,14 (m, 2H).

### 1.2. Synthesis of 1-(2,3-dihydrobenzo[b][1,4]dioxin-6-yl)-3-(5-nitroisoindol-2-yl)propan-1-one (2)

150 mg (0.91 mmol) of 5-nitroisoindoline, 276 mg (2.73 mmol) of Et₃N, 22 mg (0.18 mmol) of DMAP and 10 mL of tetrahydrofuran was mixed in a 20 mL bottle and stirred for 15 minutes. 247 mg (0.91 mmol) of 3-chloro-1-(2,3-dihydrobenzo[b][1,4]dioxin-6-yl)propan-1-one was added, and the mixture was stirred at room temperature for 4 h. The reaction was diluted with 20 ml of EA, washed with water (2×10 ml), and then with brine, and dried with sodium sulfate. The filtrate was concentrated under vacuum to obtain the crude product, which was subjected to flash column chromatography on an 80 g silica gel column with EA (ethyl acetate)/PE (petroleum) (0-80%) gradient elution to obtain 20 mg of brown solid (6.1% yield). LC-MS: RT = 1.19 min; Purity 92.0%; ESI m/z [M+1]⁺= 355.40.

### 1.3. Synthesis of 1-(3,4-dimethoxyphenyl)-3-(isoindolin-2-yl)propan-1-one (3)

To a solution of 5-bromo-2,3-dihydro-1H-isoindole (100mg, 0.5 mmol), Et₃N (150mg, 1.5 mmol), DMAP (12mg, 0.1 mmol) in 15 ml of THF was added 3-chloro -1-(2,3-dihydro-benzo[1,4]diphenyl)-propyl-1-one (114 mg, 0.5 mmol), and the mixture was stirred at room temperature for 2 hours. 10 ml of water was added to the reaction mixture, and the mixture was stirred for 10 min, and After the mixture was allowed to stand still to separate into layers, the aqueous layer was extracted once with 10 ml of EA. Then the organic layer was mixed and washed with saturated NaHCO₃ 5 ml×2 and brine 5 ml, the organic solvent was concentrated under vacuum to obtain the crude product, which was purified by flash column chromatography on a 40 g silica gel column with EA/PE gradient elution (50 -80%). In the end, 85 mg of palm oil was obtained. Yield 49.7%. LC-MS: RT = 1.31 min; Purity 95.1%; ESI m/z [M+H]⁺ = 388 FA mobile system. ¹H NMR (500MHz, DMSO-*d*₆) δ7.54-7.52 (m, 2H), 7.33(d, *J=* 8.0 Hz,2H), 7.07(d, *J =* 8.0 Hz,1H), 6.92(d, *J* = 9.0 Hz,1H),4.34-4.28 (m, 4H), 4.00(d, *J* = 17.0 Hz, 2H), 3.23 (t, *J* = 3.5 Hz, 1H).

### 1.4. Synthesis of 3-(5-chloro-1,3-dihydro-isoindol-2-yl)-1-(2,3-dihydro-benzo[1,4]dioxin-6-yl)-propan-1-one (4)

To a solution of 5-chloro-2,3-dihydro-1H-isoindole (400 mg, 2.6 mmol), Et₃N (788 mg, 7.8 mmol), and DMAP (61 mg, 0.5 mmol) in 20 ml of THF was added 3 -chloro-1-(2,3-dihydro-benzo[1,4]dioxin-6-acyl)-propyl-1-one (589 mg, 2.6 mmol). Then it was stirred at room temperature for 2 hours. 10 ml of water was added to the reaction mixture, and the mixture was stirred for 10 min, and After the mixture was allowed to stand still to separate into layers, the aqueous layer was extracted once with 10 ml of EA. Then the organic layer was mixed and washed with saturated NaHCO₃ 5 ml×2 and brine 5 ml. The organic solvent was concentrated under vacuum to obtain the crude product, which was purified by flash chromatography on a 40 g silica gel column with EA/PE gradient elution (80-100%). In the end, 120 mg of palm oil was obtained. Analysis result: yield 49.7%. LC-MS: RT = 1.25 min; Purity 95.1%, ESI m/z [M+H]⁺ = 344 FA mobile system. ¹H NMR (500MHz, DMSO-*d*₆) δ7.55-7.53 (m, 2H), 7.19-7.14 (m, 2H), 7.08 (d, *J* = 6.0 Hz,1H), 6.93-6.92 (m, 1H), 4.34-4.28 (m, 4H),4.12 (d, *J=* 7.0 Hz,4H), 3.26 (t, *J=* 4.5 Hz, 1H).

### 1.5. Synthesis of 1-(2,3-dihydrobenzo[b][1,4]dioxin-6-yl)-3-(5-methoxyisoindol-2-yl)propan-1- one (5)

100 mg (0.67 mmol) of 5-methoxyisoindolin, 203 mg (2.01 mmol) of Et₃N, 16 mg (0.134 mmol) of DMAP, and 2ml of THF were mixed in a 20 ml vial, and the mixture was stirred for 15 min. 3-chloro-1-(2,3-dihydrobenzo[b][1,4]xylene-6-acyl)-propyl-1-one 152 mg (0.67 mmol) was added, and the mixture was stirred at room temperature overnight. The reaction was stopped and concentrated under vacuum to obtain a crude product. Flash column chromatography on a 25 g silica gel column with MeOH (methanol)/DCM (dichloromethane) (0-5%) gradient elution was used to obtain 60 mg of butter. Analysis result: yield 26.4%. LC-MS: RT = 1.22 min; Purity 99.0%; ESI m/z [M+1]⁺= 340.26.

### 1.6. Synthesis of 3-(5-fluoro-1,3-dihydro-isoindol-2-yl)-1-(2,3-dihydro-benzo[1,4]diox-6-yl)-propan-1-one (6)

To a solution of 5-fluoro-2,3-dihydro-1H-isoindole (540 mg, 4.0 mmol), Et₃N (1200 mg, 12.0 mmol), DMAP (96 mg, 0.4 mmol) in 20 ml of THF was added 3- chloro-1-(2,3-dihydro-benzo[1,4]dioxin-6-yl)-propyl-1-one (891 mg, 4.0 mmol). Then it was stirred at room temperature for 2 hours. 20 ml of water was added to the reaction mixture, and the mixture was stirred for 10 min, allowed to stand still to separate into layers, and the aqueous layer was extracted once with 20 ml of EA. Then the organic layer was mixed and washed with saturated NaHCO₃ 10 ml×2 and brine 10 ml, the organic solvent was concentrated under vacuum to obtain the crude product, which was purified by flash column chromatography on a 40 g silica gel column with DCM/MeOH gradient elution (0-10%). In the end, 350 mg of palm oil was obtained. Analysis result: yield 27.2%. LC-MS: RT = 1.21 min; >95% purity ESI m/z [M+H]⁺ = 328 FA mobile system. ¹H NMR (500MHz, DMSO-*d*₆) δ7.55-7.53 (m, 2H), 7.16-7.14 (m, 1H), 6.93-6.91 (m, 3H), 4.34-4.28 (m, 4H), 4.10(d, *J=* 13.0 Hz,4H), 3.31 (t, *J=* 4.5 Hz,4H).

### 1.7. Synthesis of 3-(5-acetylisoindolin-2-yl)-1-(2,3-dihydrobenzo[b][1,4]dioxane-6-yl)propane -1-one (7)

2-isoindolin-5-yl 2-oxoethyl-1-yl 50 mg (0.32 mmol), Et₃N 98 mg (0.96 mmol), DMAP 8 mg (0.066 mmol) and 10 ml of tetrahydrofuran were mixed in a 20 ml bottle, and stirred for 30 minutes. 3-bromo-1-(2,3-dihydrobenzo[b][1,4]dioxin-6-yl)propan-1-one 86 mg (0.32mmol) was added, and the mixture was stirred at room temperature for 3 h. The reaction was stopped, concentrated under vacuum to give a crude product, and flash column chromatography on an 80 g silica gel column with methanol/dichloromethane (0-5%) gradient elution was used to obtain 20 mg butter. Analysis result: yield 18.4%. LC-MS: RT = 1.17min; Purity 99.0%; ESI m/z [M+1]⁺= 352.22.

### 1.8. Synthesis of 2-(3-(2,3-dihydrobenzo[b] [1,4]dioxin-6-yl)-3-oxopropyl)isoleucine-5-carbonitrile (8)

Using isoindole-5-carbonitrile (320 mg, 2.22 mmol), Et₃N (673 mg, 6.66 mmol), DMAP (54 mg, 0.44 mmol) as solvents, 3-chloro-1-(2,3-dihydrobenzo[1,4]dioxin-6-acyl)-propyl-1-one (503 mg, 2.22 mmol) was added in 15 ml of THF. Then it was stirred at room temperature for 4 hours. 20 ml of water was added to the reaction mixture, and the mixture was stirred for 10 min, allowed to stand still to separate into layers, and the aqueous layer was extracted once with 20 ml of EA. Then the organic layer was mixed and washed with saturated NaHCO₃ 10 ml×2 and brine 10 ml, the organic solvent was concentrated under vacuum to obtain the crude product, which was purified by flash column chromatography on a 40 g silica gel column with DCM/MeOH gradient elution (0 -10%). In the end, 230 mg of butter was obtained. Analysis result: yield 31.4%. LC-MS: RT = 1.15 min; >95% purity; ESI m/z [M+H]⁺ = 335 FA mobile system. ¹H NMR (500MHz, DMSO-*d*₆) δ7.70 (s, 1H), 7.66 (d, *J* = 7.5 Hz, 1H), 7.53 (d, *J=* 13.5 Hz, 1H), 7.49 (s, 1H), 7.44 (d, *J=* 8.0 Hz, 1H), 6.97 (d, *J=* 8.5 Hz, 1H), 4.34-4.28 (m, 4H), 3.92 (d, *J=* 15.5 Hz, 4H), 3.18 (t, *J=* 14.5 Hz,2H), 3.02 (t, *J* = 7.0 Hz, 2H).

### Example 2

### 2. Synthesis of 3-(5-aminoisoindolin-2-yl)-1-(2,3-dihydrobenzo[b][1,4]dioxin-6-yl)propan-1-one (9)

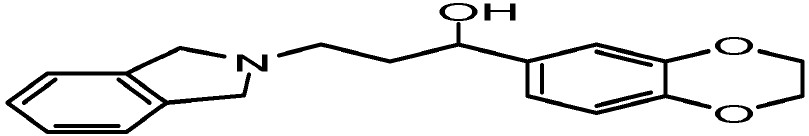

### Route 2. Synthesis of compound 9.

1-(2,3-dihydrobenzo[b][1,4]dioxin-6-yl)-3-(5-nitroisoindolin-2-yl)propan-1-one 1.8 g (5.1 mmol) and 20 ml of methanol were mixed in a 100 ml bottle and stirred for 5 minutes. 1.6 g (30.6 mmol) of NH4Cl and 0.85 g (15.2 mmol) of iron were added, the mixture was heated to reflux for 4 h, the reaction solution was cooled to room temperature, washed with methanol, and the filtrate was evaporated under reduced pressure to obtain a crude product. The crude product was purified by C18 column flash column chromatography with ACN (acetonitrile)/H2O gradient elution (5-60%) to obtain 15 mg of yellow solid. LC-MS: RT = 1.18 min; Purity 91.0%; ESI m/z [M+1]⁺= 325.29.

### Example 3

### 3. Synthesis of 1-(2,3-dihydrobenzo[b][1,4]dioxin-6-yl)-3-(isoindolin-2-yl)propan-1-ol (10)

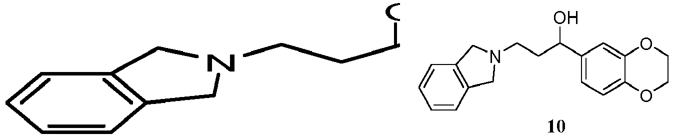

### Route 3. Synthesis of compound 10.

1-(2,3-dihydrobenzo[b][1,4]dioxin-6-acyl)-3-(isoindolin-2-acyl)propane-1 (1) 50 mg( 0.162 mmol) and 5 ml of MeOH were mixed in a 20 ml vial and stirred for 5 min, 12 mg (0.324 mmol) of NaBH₄ was added and the mixture was stirred at room temperature for 4 h, the reaction was stopped, and a crude product was obtained by concentration in vacuo. The crude product was purified by flash column chromatography on a 25 g silica gel column with methanol/dichloromethane (0-5%) gradient elution to obtain 20 mg of a brown solid. Analysis result: yield 39.8%. LC-MS: RT = 1.12 min; Purity 99.0%; ESI m/z [M+1]⁺= 312.21.

### Example 4

### 4. General preparation method of compounds 11-28.

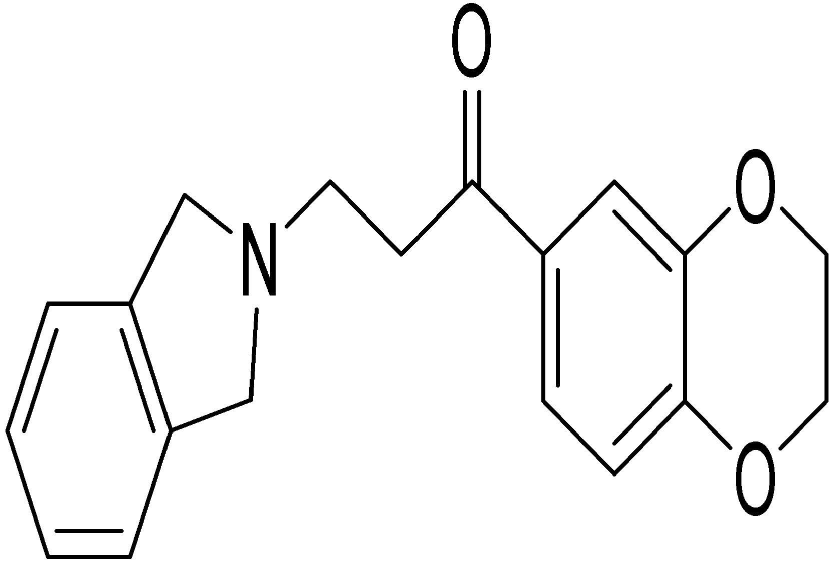

### Route 4. Synthesis of compounds 11-28.

### 4.1. Synthesis of 1-(2,3-dihydrobenzo[b][1,4]dioxin-6-yl)-3-(indolin-1-yl)propan-1-one ( 11)

To a solution of 11ii (1mmol), Et₃N (3mmol), DMAP (24mg, 0.2mmol) in 10ml of THF was added 1i (1mmol). Then it was stirred at room temperature for 2 hours. 5 ml of water was added to the reaction mixture and it was stirred for 10 min. After the mixture was allowed to stand still to separate into layers, the aqueous layer was extracted with EA 10 ml × 3. Then the organic layer was mixed and washed with saturated NaHCO₃ 5 ml×2 and brine 5 ml. The organic solvent was concentrated under vacuum to obtain the crude product, which was purified by flash chromatography on a 40 g silica gel column with EA/PE gradient elution (80-100%).

### 4.2. Synthesis of 2-(3-(2,3-dihydrobenzo[b][1,4]dioxin-6-yl)-3-oxopropyl)isoindoline-1,3-dione (12)

To a solution of 12ii (1mmol), Et₃N (3mmol), DMAP (24mg, 0.2mmol) in 10ml of THF was added 1i (1mmol). Then it was stirred at room temperature for 2 hours. 5 ml of water was added to the reaction mixture and it was stirred for 10 min. After the mixture was allowed to stand still to separate into layers, the aqueous layer was extracted with EA 10 ml × 3. Then the organic layer was mixed and washed with saturated NaHCO₃ 5 ml×2 and brine 5 ml, and the organic solvent was concentrated under vacuum to obtain a crude product, which was purified by flash column chromatography on a 40 g silica gel column with EA/PE gradient elution (80 -100%).

### 4.3. Synthesis of 1-(2,3-dihydrobenzo[b][1,4]dioxin-6-yl)-3-(3,4-dihydroisoquinoline-2(1H)-yl)propan-1-one (13)

To a solution of 13ii (1mmol), Et₃N (3mmol), DMAP (24mg, 0.2mmol) in 10ml of THF was added 1i (1mmol). Then it was stirred at room temperature for 2 hours. 5 ml of water was added to the reaction mixture and it was stirred for 10 min. After the mixture was allowed to stand still to separate into layers, the aqueous layer was extracted with EA 10 ml × 3. Then the organic layer was mixed and washed with saturated NaHCO₃ 5 ml×2 and brine 5 ml, and the organic solvent was concentrated under vacuum to obtain a crude product, which was purified by flash column chromatography on a 40 g silica gel column with EA/PE gradient elution (80-100%).

### 4.4. Synthesis of 1-(2,3-dihydrobenzo[b][1,4]dioxin-6-yl)-3-(3,4-dihydroquinoline-1(2H)-yl)propane-1-one (14)

1,2,3,4-tetrahydroquinoline 98 mg (0.738 mmol), Et₃N 120 mg (1.18 mmol), DMAP 18 mg (0.148 mmol), and THF 8 ml were mixed in a 20 ml vial. 3-bromo-1-(2,3-dihydrobenzo[b][1,4]dioxin-6-acyl)-propyl-1-one 200 mg (0.738 mmol) was added and it was stirred at room temperature for 3 h, the reaction was stopped and proceeded with the previous batch of reaction. The reaction mixture was concentrated under vacuum to obtain a crude product, which was subjected to flash column chromatography on a 40 g silica gel column with EA (ethyl acetate)/PE (petroleum) (0-30%) gradient elution to obtain 180 mg of white solid. Analysis result: yield 75.5%. LC-MS: RT = 1.84 min; 97% purity; ESI m/z [M+1]⁺=324.39.

### 4.5 Synthesis of 3-(2,3-dihydro-4H-benzo[b][1,4]oxazin-4-yl)-1-(2,3-dihydrobenzo[b][1,4]dioxin-6-yl))propan-1-one (15)

15ii (1mmol), Et₃N (3mmol), DMAP (24mg, 0.2mmol) were added to a solution of 10ml of THF, and 1i (1mmol) was added. Then it was stirred at room temperature for 2 hours. 5 ml of water was added to the reaction mixture and it was stirred for 10 min. After the mixture was allowed to stand still to separate into layers, the aqueous layer was extracted with EA 10 ml × 3. Then the organic layer was mixed and washed with saturated NaHCO₃ 5 ml×2 and brine 5 ml. The organic solvent was concentrated under vacuum to obtain a crude product, which was purified by flash chromatography on a 40 g silica gel column with EA/PE gradient elution (80-100% ).

### 4.6 Synthesis of 1-(2,3-dihydrobenzo[b][1,4]dioxin-6-yl)-3-(pyrrolidin-1-yl)propan-1-one (16)

31.4 mg (0.442 mmol) of pyrrolidone, 134.1 mg (1.326 mmol) of Et₃N, 11 mg (0.084 mmol) of DMAP, and 4 ml of THF were mixed in a 20 ml vial. 3-chloro-1-(2,3-dihydrobenzo[b][1,4]dioxin-6-yl)-propyl-1-one 100 mg (0.442 mmol) was added. The mixture was then stirred at room temperature for 2 hours. The reaction was stopped, and a crude product was obtained by concentration. The crude product was purified by flash column chromatography on a 25 g silica gel column with MeOH (methanol)/DCM (dichloromethane) gradient elution (0-3%). 40 mg of yellow solid were obtained. Analysis result: yield 34.7%. LC-MS: RT = 1.26 min; Purity: 99.5%; ESI m/z [M+1]⁺=262.32.

### 4.7. Synthesis of 1-(3-(2,3-dihydrobenzo[b][1,4]dioxin-6-yl)-3-oxopropyl)pyrrolidin-3-one ( 17)

Pyrrolidin-3-one hydrochloride 116 mg (0.738 mmol), Et₃N 224 mg (2.214 mmol), DMAP 18 mg (0.148 mmol) and 8 ml of tetrahydrofuran were mixed in a 20 ml bottle and stirred for 15 minutes. 3-bromo-1-(2,3-dihydrobenzo[b][1,4]dioxin-6-yl)propyl-1-one 200 mg (0.738 mmol) was added, and it was stirred at room temperature for 4 h. The reaction was stopped and concentrated to obtain a crude product, which was purified by flash column chromatography on a 120 g silica gel column with MeOH (methanol)/DCM (dichloromethane) (0-10%) gradient elution to obtain 60 mg of white solid. Analysis result: yield 29.6%. LC-MS: RT = 0.95min; 90.0% purity; ESI m/z [M+1]⁺=276.30.

### 4.8. Synthesis of 1-(3-(2,3-dihydrobenzo[b][1,4]dioxin-6-yl)-3-oxopropyl)-5-(4-fluorophenyl)pyridine-2(1H)-one (18)

5-(4-fluorophenyl)-1H-pyridine-2-1 (163 mg, 0.9 mmol), Et₃N (270 mg, 2.7 mmol), DMAP (24 mg, 0.2 mmol) -chloro-1-(2,3-dihydro-benzo[1,4]dioxin-6-yl)-propyl-1-one (204 mg, 0.9 mmol) were added to 10ml of THF solution. Then it was stirred at room temperature for 4 hours. 20 ml of water was added to the reaction mixture, and it was stirred for 10 min, allowed to stand still to separate into layers, and the aqueous layer was extracted once with 20 ml of EA. Then the organic layers were combined, washed with 10 ml×2 of NaHCO₃ and 10 ml of brine. The solvent was removed in vacuo to obtain a crude product, which was purified by flash column chromatography on a 40 g silica gel column with methanol/DCM (0-10%) gradient elution. In the end, 60 mg of colorless oil was obtained. Analysis result: yield 18.4%. LC-MS: RT = 1.59 min; >95% purity; ESI m/z [M+Na]⁺ = 402 FA mobile system. ¹H NMR (400MHz, DMSO-*d*₆) δ7.79 (d, *J* = 2.4 Hz,1H), 7.56-7.53 (m, 1H), 7.49-7.45 (m, 2H), 7.37-7.36 (m, 2H), 7.11-7.07 (m, 2H), 6.86(d, *J* = 8.4 Hz,1H), 6.62(d, *J* = 9.6 Hz, 1H), 4.38 (t, *J* = 5.6Hz, 4H), 4.37-4.27 (m, 2H), 4.25-4.23 (m, 2H), 3.50-3.43 (m, 2H).

### 4.9. Synthesis of 1-(2,3-dihydrobenzo[b][1,4]dioxin-6-yl)-3-(pyrimidin-2-yl)piperidin-1-yl)pyrrolidine-1-one (19)

2-(piperazin-1-yl)pyrimidine (180 mg, 1.10 mmol), Et₃N (333 mg, 3.30 mmol), DMAP (27 mg, 0.22 mmol) were added to a solution of 15ml THF, and 3-chloro-1 -(2,3-dihydrobenzo[1,4]dioxin-6-yl)-propyl-1-one (249 mg, 1.10 mmol) was added. Then it was stirred at room temperature for 4 hours. 20 ml of water was added to the reaction mixture, and it was stirred for 10 min, allowed to stand still to separate into layers, and the aqueous layer was extracted once with 20 ml of EA. Then the organic layer was mixed and washed with saturated NaHCO₃ 10 ml×2 and brine 10 ml, the organic solvent was concentrated under vacuum to obtain a crude product, which was purified by flash column chromatography on a 40 g silica gel column with DCM/MeOH gradient elution (0 -10%). In the end, 240 mg of yellow oil was obtained. Analysis result: yield 61.9%. LC-MS: RT = 1.01 min; >95% purity; ESI m/z [M+H]⁺=355 FA mobile system. ¹H NMR (500 MHz, DMSO-*d*₆) δ 8.31 (d, *J* = 4.5 Hz, 2H), 7.52-7.51 (m, 2H), 6.92-6.91 (m, 1H), 6.49 (t, *J* = 4.5 Hz, 1H), 4.33-4.28 (m, 4H), 3.87 (s, 4H), 3.19 (t, *J=* 7.5 Hz, 2H), 2.91 (t, *J=* 7.5 Hz, 2H), 2.62 (t, *J=* 4.5 Hz, 4H).

### 4.10. Synthesis of 1-(2,3-dihydrobenzo[b][1,4]dioxin-6-yl)-3-(isoquinolin-6-ylamino)propan-1-one ( 20)

3-bromo-1-(2,3-dihydrobenzo[b][1,4]dioxin-6-acyl)-propyl-1-one 281mg (1.04 mmol), isoquinoline-6-amine, 100 mg (0.69 mmol), NaI 104 mg (0.69 mmol) K₂CO₃ 200 mg (1.38 mmol), DMF/H₂O were mixed in a 20 ml vial. It was stirred at room temperature for 2 hours. No reaction was detected by thin layer chromatography, and the reaction mixture was heated to 70°C and stirred overnight. The reaction was stopped and concentrated to obtain a crude product, which was purified by flash column chromatography on a 40 g silica gel column with MeOH (methanol)/DCM (dichloromethane)(0-3%) gradient elution to obtain 20 mg of a yellow solid. LC-MS: RT = 1.22 min; Purity: 95.0%; ESI m/z [M+1]+=335.22. ¹H NMR (500 MHz, DMSO-*d*₆): δ 8.86 (s, 1H), 8.17 (d, *J* = 5.5 Hz, 1H), 7.74 (d, *J* = 9.0 Hz, 1H), 7.53-7.45 (m, 3H), 7.07-7.05 (m, 1H), 6.98 (d, *J* = 8.0 Hz, 1H) ,6.69-6.62 (m, 2H), 4.33-4.28 (m, 5H), 3.51-3.48 (m, 3H).

### 4.11. Synthesis of 1-(2,3-dihydro-benzo[1,4]diox-6-yl)-3-(3-phenyl-piperidin-1-yl)-propan-1-one (21)

To a solution of 3-phenylpiperidine (400 mg, 2.5 mmol), Et₃N (750 mg, 7.5 mmol), DMAP (61 mg, 0.5 mmol) in 15ml of THF was added 3-chloro-1-(2,3-dihydro-benzo[1,4]dioxin-6-acyl)-propyl-1-one (561 mg, 2.5 mmol). Then it was stirred at room temperature for 2 hours. 20 ml of water was added to the reaction mixture, and it was stirred for 10 min, and allowed to stand still to separate into layers, and the aqueous layer was extracted once with 20 ml of EA. Then the organic layer was mixed and washed with saturated NaHCO₃ 10 ml×2 and brine 10 ml, the organic solvent was concentrated under vacuum to obtain a crude product, which was purified by flash column chromatography on a 40 g silica gel column with MeOH/DCM gradient elution (0-10%). In the end, 280 mg of palm oil was obtained. Analysis result: yield 34.7%. LC-MS: RT = 1.31 min; >95% purity; ESI m/z [M+H]⁺ = 352 FA mobile system. ¹H NMR (500MHz, DMSO-*d*₆) δ7.51-7.49 (m, 2H), 7.32-7.29 (m, 2H), 7.24-7.22 (m, 3H), 6.91-6.89 (m, 1H), 4.32-4.26 (m, 4H), 3.23 (s, 2H),3.11 (t, *J=* 14.5 Hz,2H), 2.94 (s, 3H),2.20 (s, 2H), 1.96(d, *J* = 13.0 Hz,1H), 1.84 (s, 2H), 1.19-1.17 (m, 1H).

### 4.12. Synthesis of 3-(3-(2,3-dihydrobenzo[b][1,4]dioxin-6-yl)-3-oxopropyl)-2-thiothiazolidine-4-one (22)

2-thiophenthiazolidinone-4- 1,61 mg (0.42 mmol) and 1-(2,3-dihydrobenzo[b][1,4]dioxin-6-yl)prop-2-en-1-one 80 mg (0.42 mmol) were mixed in a 20 ml vial, and stirred at 110°C for 1 hour. After the reaction stopped, the title compound was purified by flash column chromatography on a 60g C18 column with ACN (acetonitrile)/H₂O (5-95%) gradient elution. In the end, 20 mg of yellow solid was obtained. Analysis result: yield 12.4%. LC-MS: RT = 1.57 min; Purity 91.0%; ESI m/z [M-1]⁺= 321.93.

### 4.13. Synthesis of tert-butyl ((1-(3-(2,3-dihydrobenzo[b][1,4|dioxin-6-yl)-3-oxopropyl)-3-hydroxyazetidin-3-yl)methyl)carbamate (23)

To a solution of tert-butyl ((3-hydroxyazetidine-3-acyl)methyl)carbamate (202 mg, 1.0 mmol), Et₃N (303 mg, 3.0 mmol), DMAP (25 mg, 0.2 mmol) in 10ml of THF was added 3-bromo-1-(2,3-dihydrobenzo[1,4]dioxin-6-acyl)-propyl-1-one (271 mg, 1.0 mmol). Then it was stirred at room temperature for 2 hours. 10 ml of water was added to the reaction mixture, and it was stirred for 10 min, and allowed to stand still to separate into layers, and the aqueous layer was extracted once with 20 ml of EA. Then the organic layers were combined, and washed with 10 ml×2 of NaHCO₃ and 10 ml of brine. The solvent was removed in vacuo to obtain the crude product, which was purified by flash column chromatography on a 40 g silica gel column with methanol/DCM (0-10%) gradient elution. In the end, 260 mg of white solid was obtained. Analysis result: yield 66.3%. LC-MS: RT = 1.22 min; >95% purity ESI m/z [M+H]⁺ = 393 FA mobile system.

¹H NMR (500MHz, DMSO-*d*₆) δ7.50-7.48 (m, 2H), 6.91(d, *J* = 7.2 Hz, 1H), 4.33-4.29 (m, 6H), 3.87-3.82 (m, 4H), 3.65-3.55 (m, 2H), 3.50-3.47 (m, 2H).

### 4.14. Synthesis of 1-(2,3-dihydrobenzo[b][1,4]dioxin-6-yl)-3-(6-methoxy-2H-indazol-2-yl)propan-1-one (24)

((2h-indole-5-acyl)oxy)methyl 127 mg (0.67 mmol) and 1-(2,3-dihydrobenzo[b][1,4]dioxin-6-yl)prop-2-en-1-one 127 mg (0.67 mmol) were mixed in a 20 ml vial, and stirred at 110°C for 1 hour. The compound was purified by flash column chromatography on a 60 g C18 column with ACN (acetonitrile)/H₂O (5-95%) gradient elution. In the end, 30 mg of yellow solid was obtained. Analysis result: yield 19.6%. LC-MS: RT = 1.54 min; Purity 99.0%; ESI m/z [M+1]⁺= 339.26.

### 4.15. Synthesis of 1-(2,3-dihydrobenzo[b][1,4]dioxin-6-yl)-3-(1H-pyrazolo[3,4-c]pyridin-1-yl)propan-1-one (25)

1-(2,3-dihydrobenzo[b][1,4]dioxin-6-acyl)prop-2-en-1- 1 160 mg (0.84mmol), 1h - pyrazoline[3,4-c]pyridine 100 mg (0.84 mmol) were mixed in a 20 ml vial. It was then stirred at 110°C for 2 hours. The reaction mixture was purified by flash column chromatography on a 40 g silica gel column with DCM/MeOH (0-5%) gradient elution to obtain 20 mg of a white solid. Analysis result: yield 7.69%. LC-MS: RT = 1.11 min; Purity: 97.0%; ESI m/z [M+1]+=310.25.1HNMR (400 MHz, CDC13): δ 7.43(s, 1H), 7.28 (d, *J* = 6.0Hz, 1H), 6.80 (d, *J* = 5.6Hz, 1H), 6.62-6.60 (m, 2H), 6.40 (s, 1H), 6.03 (d, *J* = 9.2Hz, 1H), 4.13(t,J=6Hz, 2H), 2.86(t,J=6Hz, 2H), 0.469-0.389(m, 4H).

### 4.16. Synthesis of 3-((1H-isoindol-3-yl)amino)-1-(2,3-dihydrobenzo[b][1,4]dioxan-6-yl)propane -1-one (26)

1H-isoindole-3-amine hydrochloride 150 mg (0.885 mmol), Et₃N 270 mg (2.655 mmol), DMAP 21 mg (0.177 mmol) and 2 ml of tetrahydrofuran were mixed in a 20 ml bottle and stirred for 30 minutes. 3-bromo-1-(2,3-dihydrobenzo[b][1,4]dioxen-6-yl)propan-1-one 240 mg (0.885mmol) was added, and it was stirred at room temperature for 4 h. It was conncentrated to obtain a crude product, which was purified by flash column chromatography on a 40 g C18 column with ACN (acetonitrile)/H₂O (0-40%) gradient elution to obtain 100 mg of brown solid. Analysis result: yield 26.2%.

LC-MS: RT = 1.23 min; Purity 90.0%; ESI m/z [M+1]⁺ =323.25.

### 4.17. Synthesis of 1-(3-(2,3-dihydrobenzo[b][1,4]dioxin-6-yl)-3-oxopropyl)-2,3-dihydroquinoline-4(1H)-one (27)

2,3-dihydroquinolin-4(1H)-one 100mg (0.68 mmol) and 1-(2,3-dihydrobenzo[b][1,4]dioxin-6-yl)prop-2-en-1-one 129 mg (0.68 mmol) were mixed in a 20 ml vial, and stirred at 110°C for 1 h. After the reaction stopped, a flash column chromatography with 60 g C18 column with ACN (acetonitrile)/H2O gradient elution (5-95%) was used to finally obtain 40 mg of yellow solid. Analysis result: yield 26.0%.

LC-MS: RT = 1.59 min; Purity 95.0%; ESI m/z [M+1]⁺= 338.34.

### 4.18. Synthesis of 1-(2,3-dihydrobenzo[b][1,4]dioxin-6-yl)-3-(4-phenyl-1H-pyrazol-1-yl)propan-1-one (28)

4-phenyl-1H-pyrazole 61 mg (0.42 mmol), 1-(2,3-dihydrobenzo[b][1,4]dioxin-6-yl)prop-2-en-1-one 80 mg (0.42 mmol) were mixed in a 20 ml bottle and stirred at 140 oC for 30 minutes. The reaction was stopped and sent to a flash column chromatography on a 60 g C18 column with ACN (acetonitrile)/H₂O (0-5%) gradient elution to purify the title compound. In the end, 30 mg of white powder was obtained. Analysis result: yield 21.2%. LC-MS: EPN18027-081-A RT = 1.65 min; Purity 95.0%; ESI m/z [M+1]⁺= 335.22.

### Example 5

### 5. Synthesis of 2-(3-(2,3-dihydrobenzo[b][1,4]dioxin-6-yl)propyl)isoindoline (29)

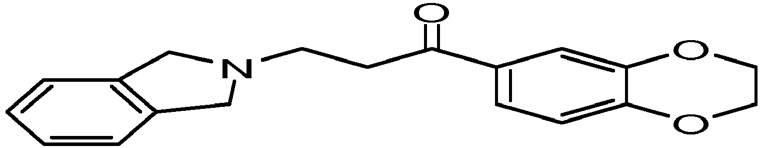

### Route 5. Synthesis of compound 29.

100 mg (0.323 mmol) of compound 1 and 1 ml of TFA were mixed in a 10 ml vial. The mixture was cooled to 10°C. 150 mg (1.3 mmol) of triethylsilane was added dropwise. The mixture was stirred at room temperature overnight. The reaction stopped. The mixture was concentrated under vacuum to obtain a crude product. The crude product was purified using 40 g C18 column flash column chromatography with ACN (acetonitrile)/H₂O (0-40%) gradient elution method. In the end, 20 mg of butter was obtained. Analysis result: yield 21.0% LC-MS: RT = 1.87 min; purity 95.0%; ESI m/z [M+1]+=296.20.

### Example 6

### 6. General preparation method of compounds 30-38.

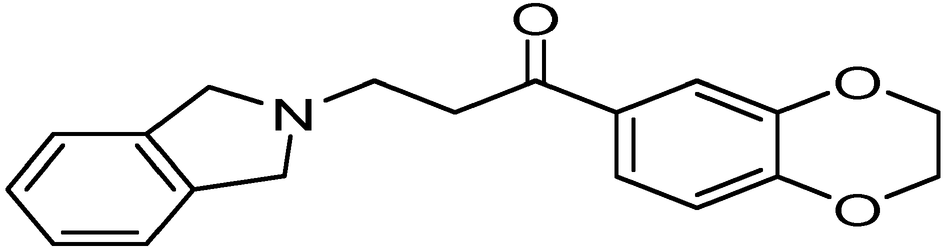

### Route 6. Synthesis of compounds 30-38.

### 6.1. Synthesis of 3-(isoindolin-2-yl)-1-(1,2,3,4-tetrahydroquinolin-7-yl)propan-1-one (30)

700 mg (4.5 mmol) of isoindoline hydrochloride, 400 mg (13.5 mmol) of Cs₂CO₃, and ACN (10 ml)+H₂O (0.5ML) were mixed and stirred in a 50 ml vial. After 15 minutes, 30i (4.5 mmol) was added and the mixture was stirred overnight at 50°C. The reaction was stopped, poured into water, extracted into CH₂Cl₂ 25ml × 3, and washed with 25ml × 2 water. The organic layer was dried on Na₂SO₄. After filtration, the solvent was removed to obtain a yellow solid, which was purified by silica gel column chromatography and eluted with MeOH/DCM=0-5% to obtain 700 mg of 3-(isoindolin-2-yl)-1-(1,2,3,4-tetrahydroquinolin-7-yl)-propan-1-one. LC-MS: RT = 1.22 min; ESI m/z [M+1]+=307.25.

### 6.2. Synthesis of 1-(2,3-dihydrobenzo[b][1,4]dioxin-6-yl)-4-(isoindolin-2-yl)butan-1-one (31)

97.3 mg (0.625 mmol) of isoindoline hydrochloride, 611 mg (1.875 mmol) of Cs₂CO₃, 93.75 mg (0.625 mmol) of NaI, and ACN (6ml) were mixed and stirred in a 20 ml vial. After 15min, 4-bromo-1-(2,3-dihydrobenzo[b][1,4]dioxin-6-acyl)butane-1-one, 178 mg (0.625 mmol) was added, and the mixture was stirred overnight at 80°C. The reaction was stopped, poured into water, extracted with CH₂Cl₂ 25ml × 3, washed with 25ml × 2 water, and dried with Na₂SO₄. After filtration, the solvent was concentrated into a white solid, and purified by silica gel column chromatography and eluted with MeOH/DCM=0-5% to obtain 120 mg of a white solid. LC-MS: RT = 1.23 min; 96.1% purity; ESI m/z [M+1]⁺=324.25. ¹H NMR (500 MHz, CDCl₃): δ 7.53-7.51 (m ,4H), 6.92-6.90 (m, 2H), 4.33-4.27 (m, 5H), 3.74-3.72 (m, 4H), 3.07-3.04 (m, 4H), 2.02-1.97 (m, 4H).

### 6.3. Synthesis of 1-(2,3-dihydrobenzo[b][1,4]dioxin-6-yl)-2-(isoindolin-2-yl)ethan-1-one (32)

78 mg (0.5 mmol) of isoindoline hydrochloride, 151 mg (1.5 mmol) of Et₃N, 12 mg (0.1 mmol) of DMAP, and 10 ml of THF were mixed in a 20 ml vial, and stirred for 15 min. 3-bromo-1-(2,3-dihydrobenzo[b][1,4]dioxin-6-acyl)-propyl-1-one 128 mg (0.5 mmol) was added, and the mixture was stirred at room temperature overnight. The reaction was stopped, and a crude product was obtained by vacuum concentration. The crude product was purified by flash column chromatography on a 40 g silica gel column with EA (ethyl acetate)/PE (petroleum) gradient elution (0-30%). In the end, 20 mg brown semi-solid was obtained. Analysis result: yield 13.6%. LC-MS: RT = 1.18 min; Purity 91.0%; ESI m/z [M+1]⁺= 296.38.

### 6.4. Synthesis of (2,3-dihydrobenzo[b][1,4]dioxin-6-yl)(isoindolin-2-yl)methanone (33)

2,3-dihydrobenzo[b][1,4]dioxin-6-carboxylic acid (200mg, 1.11 mmol) and triethylamine (336.3 mg 3.33 mmol) were dissolved in CH₂Cl₂ (3ml) and the mixture was stirred for 30 min and HATU (505.4 mg 1.33 mmol) was added. Then isoindoline hydrochloride (206 mg, 1.33 mmol) was added. After 2 hours, 10ml saturated aqueous NaCl was added, and the product was extracted with CH₂Cl₂ 10ml×3. The organic layer was mixed and washed with 5% NaHCO₃, saturated aqueous NaCl 10ml, and the organic layer was concentrated to obtain a crude product, and silica gel column chromatography with elution was performed to obtain a crude product. LCMS: RT = 1.53 min; Purity: 97.3%; ESI m/z [M+1]⁺=282.20.1H NMR (500 MHz, DMSO-d6): δ 7.39-7.38 (m, 1H), 7.30-7.28 (m, 3H), 7.14-7.11 (m, 2H), 6.94-6.93 (m, 1H), 4.82 (d, *J* = 13.0Hz, 4H), 4.31-4.28 (m, 4H).

### 6.5. Synthesis of 1-(2,3-dihydrobenzofuran-5-yl)-3-(isoindolin-2-yl)propan-1-one (34)

700 mg (4.5 mmol) of isoindoline hydrochloride, 400 mg (13.5 mmol) of Cs₂CO₃, and ACN (10 ml) + H₂O (0.5 mL) were mixed and stirred in a 50 ml vial. After 15 minutes, 34i (4.5 mmol) was added and the mixture was stirred at 50°C overnight. The reaction was stopped, poured into water, extracted into CH₂Cl₂ 25ml × 3, and washed with 25ml × 2 water. The organic layer was dried on Na₂SO₄. After filtration, the solvent was removed to obtain a yellow solid, which was purified by silica gel column chromatography and eluted with MeOH/DCM=0-5% to obtain 700 mg of 3-(isoindolin-2-yl)-1-(1,2,3,4-tetrahydroquinolin-7-yl)propyl-1-one.

### 6.6. Synthesis of 1-(benzo[d][1,3]dioxol-5-yl)-3-(isoindolin-2-yl)propan-1-one (35)

Isoindole hydrochloric acid (146 mg, 0.94 mmol), Et₃N (285 mg, 2.82 mmol), DMAP (23 mg, 0.19 mmol) 1-(benzo[d][1,3]dioxapyrimidine-5-acyl-3-chloropropyl-1-one (200 mg, 0.94 mmol) were added to 10ml of THF, and the mixture was stirred at room temperature for 2 hours. The reaction mixture was a brown solution. The results of LCMS detection were as follows: 5 ml of water was added to the reaction mixture and the mixture was stirred for 10 min. After the mixture was allowed to stand still to separate into layers, the aqueous layer was extracted once with 10 ml of EA. Then the organic layer was mixed and washed with saturated NaHCO₃ 5 ml×2 and brine 5 ml, and the organic solvent was concentrated under vacuum to obtain a crude product, which was purified by flash column chromatography on a 25 g silica gel column with EA/PE gradient elution (50-80%). In the end, 130 mg of a dark solid was obtained. Analysis result: yield 46.7%. LC-MS: RT = 1.73 min; 99% purity; ESI m/z [M+H]⁺=296 BASE mobile system. ¹H NMR (400MHz, DMSO-*d*₆) δ7.59 (d, *J* = 8.0Hz, 1H), 7.45 (s, 1H), 7.18 (s, 4H), 6.85 (d, *J* = 8.0 Hz, 1H), 6.03 (s, 2H), 4.00 (s, 4H), 3.21-3.19 (m,4H).

### 6.7. Synthesis of 6-(3-(isoindolin-2-yl)propionyl)chroman-4-one (36)

Isoindolin hydrochloride 260.5 mg (1.68 mmol), Et₃N 510 mg (5.04 mmol), DMAP 41 mg (0.34 mmol) and THF (6 ml) were mixed in a 20 ml vial. After stirring for 15 min, 400 mg (1.68 mmol) of 3-chloro-1-(2,3-dihydrobenzo[b][1,4]dioxin-6-acyl)-propyl-1-one was added. Then it was stirred at room temperature for 2 hours, and the reaction was stopped. The reaction mixture was concentrated under vacuum to obtain a crude product. The residue was purified by silica gel column chromatography eluted with dcm / MeOH=0-5% to obtain 155 mg of 6-(3-(isoindolin-2-yl)propionyl)chroman-4-one LCMS: RT = 1.16 min; Purity 98.0%; ESI m/z [M+1]⁺=322.17. ¹H NMR (500 MHz, CDCl₃): δ 8.53 (d, *J* = 2.5 Hz, 1H), 8.17-8,15 (m, 1H), 7.20 (s, 4H), 7.06 (d, *J* = 9.0 Hz, 1H), 4.62 (t, *J* = 6.5 Hz, 2H), 4.05 (s, 4H), 3.32-3.22 (m, 4H), 2.88 (t, *J=* 6.5 Hz, 2H).

### 6.8. Synthesis of 1-(2,3-dihydrobenzo[b][1,4]oxathiol-6-yl)-3-(isoindolin-2-yl)propan-1-one (37)

Isoindoline hydrochloride 77.17 mg (0.50 mmol), Et₃N 125 mg (1.24 mmol), KI 82 mg (0.5 mmol), and acetone (6 ml) were mixed in a 20 ml vial, and stirred for 15 min, and 3-chloro-1-(2,3-dihydrobenzo[b][1,4]oxfloxacin-6-acyl)-propyl-1-one 100 mg (0.41 mmol) was added to the reaction mixture, and it was continuously stirred overnight at room temperature. The reaction stopped. The reaction mixture was concentrated under vacuum to obtain a crude product, which was purified by silica gel column chromatography eluted with DCM/MeOH=0-5% to obtain 40 mg of pink solid. LC-MS: RT = 1.27 min; Purity 95.0%; ESI m/z [M+1]⁺ =326.25. ¹HNMR (500 MHz, CDCl₃): δ 7.75-7.65 (m, 1H), 7.50-7.45 (m, 1H) ,7.23-7.20 (m, 4H), 7.14-7.13(m, 0.5H), 6.89-6.87 (m, 0.5H), 4.50-4.43 (m, 2H), 4.15 (d, *J* = 9.0 Hz, 4H), 3.33 (t, *J=* 4.5Hz, 4H), 3.19-3,14 (m, 2H).

### 6.9. Synthesis of 1-(3,4-dimethoxyphenyl)-3-(isoindolin-2-yl)propan-1-one (38)

Hydrochloride (135 mg, 0.87 mmol), Et₃N (264 mg, 2.61 mmol), DMAP (21 mg, 0.17 mmol) 3-chloro-1-(2,3-dihy drobenzo [b][1,4]dioxin-6-acyl)-propyl-1-one (200 mg, 0.5 mmol) were added to 10ml of THF, and the mixture was stirred at room temperature for 2 hours. The reaction mixture was a brown solution, and the detection by LC-MS was as follows. 5 ml of water was added to the reaction mixture and the mixture was stirred for 10 min. After the mixture was allowed to stand still to separate into layers, the aqueous layer was extracted once with 10 ml of EA. Then the organic layer was mixed and washed with saturated NaHCO₃ 5 ml×2 and brine 5 ml, and the organic solvent was concentrated under vacuum to obtain a crude product, which was purified by flash column chromatography with silica gel column 25 g with EA/PE gradient elution (50 -80%). In the end, 135 mg of brown solid was obtained. Analysis result: yield 47.8%. LC-MS: RT = 1.44 min; purity 99%; ESI m/z [M+H]⁺= 312 BASE mobile system. ¹H NMR (400MHz, DMSO-*d*₆) δ7.63 (d, *J* = 10.2 Hz, 1H), 7.54 (d, *J* = 2.0 Hz, 1H), 7.18 (s, 4H), 6.89 (d, *J =* 8.4 Hz, 1H), 4.01 (s, 4H), 3.93 (d, *J =* 10.2 Hz,5.6H), 3.26-3.20 (m, 4H).

### Example 7

### 7. Synthesis of compound 39.

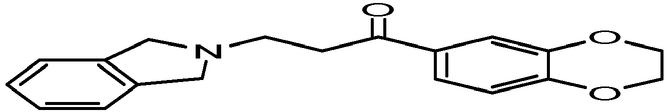

### Route 7. Synthesis of compound 39.

1-(3,4-dimethoxyphenyl)-3-(isoindol-2-yl)-propyl-1-one (80 mg, 0.14 mmol) was added to 10ml of DCM, and BBr₃ ( 195 mg, 0.78 mmol) was added dropwise, and the mixture was stirred overnight at room temperature. The reaction mixture was a brown solution, and the detection by LC-MS was as follows. The reaction was processed in conjunction with EPN18034-008-1. The mixture was quenched by adding methanol dropwise. The organic solvent was concentrated under vacuum to obtain a crude product, which was purified by flash column chromatography on a 25 g silica gel column with EA/PE gradient elution (50-80%). In the end, 40 mg of dark solid was obtained. Analysis result: yield 35.4%. LC-MS: RT = 1.93 min; 95% purity; ESI m/z [M+H]⁺=284 BASE mobile system. ¹H NMR (500MHz, DMSO-*d*₆) δ7.53 (dd, *J=* 2.0, 8.5Hz, 1H), 7.49 (d, *J=* 2.0 Hz, 1H), 7.41 (d, *J=* 2.0 Hz, 4H), 6.88 (d, *J* = 8.5 Hz, 1H),4.96 (d, *J* = 13.5 Hz, 2H),4.67 (d, *J* = 13.5 Hz, 2H),3.85 (t, *J* = 6.5 Hz,2H), 3.61 (t, *J* = 6.5 Hz,2H).

### Example 8

### 8. Methods of preparing compounds 40 and 41.

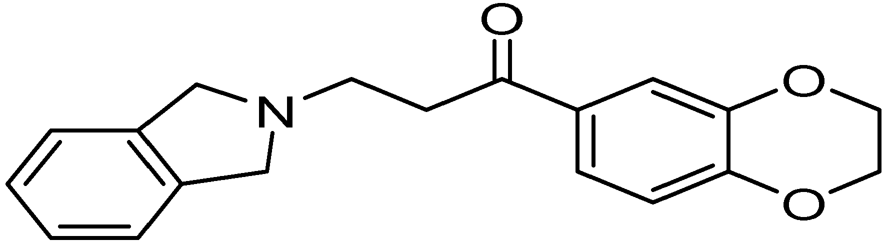

### Route 8. Synthesis of compounds 40, 41.

### 8.1.1 Synthesis of 5-bromoisoindoline-1,3-dione (40a)

Urea (53 mg, 0.88 mmol) was added to a solution of 5-bromoisobenzofuran-1,3-dione in 2 ml of DMF. It was then stirred at 130°C for 3 hours. The reaction mixture was a colorless solution, and 10ml of water was added to stop the reaction. After the mixture was allowed to stand still to separate into layers, the aqueous layer was extracted with EA 6ml×2. Then the organic layer was mixed and washed with saturated NaHCO₃ 5ml×2 and brine 5ml, and the organic solvent was concentrated under vacuum. The crude product 100mg was a pale yellow solid without the need for further purification. LC-MS: RT = 1.35 min; Purity 90%; ESI m/z [M-H]⁺ =224 FA mobile system.

### 8.1.2 Synthesis of 5-bromoisoindoline (40b)

5-bromoisoindole-1,3-dione (4 g, 17.7 mmol) was added to 120 ml of BH3/THF, overnight at 70°C, and the reaction was stopped by adding 10 ml of MeOH dropwise. The mixed solution was concentrated under vacuum, and the blend was added. 40 ml of hydrochloride was added, and the mixture was stirred at 70°C for 4 hours. Then 1M NaOH aq was added to adjust the pH to 14. After static separation of layers, the aqueous layer was extracted with DCM 100 ml×3. Then the organic layer was mixed and washed with brine 50ml×2, water 50ml, and the organic solvent was concentrated under vacuum to obtain 2.0 g of crude product. LC-MS: RT = 0.95 min; ESI m/z [M+H]⁺=198 FA mobile system.

### 8.1.3 Synthesis of 5-bromoisoindoline (40c)

To a solution of 5-bromo-2,3-dihydro-1H-isoindole (1.8 g, 9.0 mmol) was added Boc₂O (4.0 mg, 18.0 mmol) Na₂CO₃ aq 10ml in THF 40ml. Then it was stirred at room temperature for 4 hours. Then 30ml of water was added to the reaction mixture and the mixture was stirred for 10 min. After the mixture was allowed to stand still to separate into layers, the aqueous layer was extracted with EA 30ml×3. Then the organic layer was mixed and washed with saturated NaHCO₃ 20 ml×2 and brine 20 ml, the organic solvent was concentrated under vacuum to obtain a crude product, which was purified by flash column chromatography on a 40 g silica gel column with PE/EA gradient elution (10 -30%). A yellow solid (1.6 g, yield 59.2%) was finally obtained.

LC-MS: RT = 1.87 min; ESI m/z [M+H]⁺=299 FA mobile system.

### 8.1.4 Synthesis of tert-butyl 5-(4-fluorophenyl)isoindoline-2-carboxylate (40e)

Tert-butyl 5-bromoisoindole-2-carboxylate (300 mg, 1.00 mmol), K₃PO₄ (535 mg, 2.52 mmol), Pd(dppf)Cl2 (146 mg, 0.20 mmol) and 2-(4 -fluorophenyl)-4,4,5,5-tetramethyl-1,3,2-dioxborane (444 mg, 2.00 mmol) were stirred in 1,4-dioxborane/H₂O 9.6 ml for 2 hours. The reaction was stopped, and concentration was performed under vacuum to obtain a crude product, and the obtained crude product was purified by flash column chromatography on a 40 g silica gel column with MeOH/DCM gradient elution (0-10%). A white solid (275 mg, yield 68.2%) was finally obtained. LC-MS: RT = 1.95 min; ESI m/z [M+H]⁺=314 FA mobile system.

### 8.1 Synthesis of 1-(2,3-dihydrobenzo[b][1,4]dioxin-6-yl)-3-(5-(4-fluorophenyl)isoindolin-2-yl)propan-1-one (40)

To a solution of 5-(4-fluorophenyl)isoindolin (187 mg, 0.88 mmol), Et₃N (267 mg, 2.64 mmol), DMAP (22 mg, 0.18 mmol) in 15ml of THF was added 3-chloro- 1-(2,3-dihydrobenzo[1,4]dioxin-6-yl)-propyl-1-one (200 mg, 0.88 mmol). Then it was stirred at room temperature for 4 hours. 20 ml of water was added to the reaction mixture, and the mixture was stirred for 10 min, allowed to stand still to separate into layers, and the aqueous layer was extracted once with 20 ml of EA. Then the organic layer was mixed and washed with saturated NaHCO₃ 10 ml×2 and brine 10 ml. The organic solvent was concentrated under vacuum to obtain a crude product, which was purified by flash column chromatography on a 40 g silica gel column with MeOH/DCM gradient elution (0-10%). In the end, 230 mg of a red solid was obtained. Analysis result: yield 69.3%. LC-MS: RT = 1.43 min; >95% purity; ESI m/z [M+H]⁺ = 404 FA mobile system. ¹H NMR (400MHz, DMSO-*d*₆) δ 7.55-7.52 (m, 2H), 7.50-7.46 (m, 2H), 7.38-7.35 (m, 2H), 7.26-7.24 (m, 1H), 7.11-7.07 (m, 2H), 6.90(d, *J* = 8.8 Hz, 1H), 4.32-4.26 (m, 4H), 4.13 (d, *J* = 4.0 Hz,4H), 3.30 (s, 4H).

### 8.2.1 Synthesis of tert-butyl 5-(pyridin-4-yl)isoindoline-2-carboxylate (41e)

Tert-butyl 5-bromoisoindole-2-carboxylate (250 mg, 0.84 mmol), K₃PO₄ (535 mg, 2.52 mmol), Pd(dppf)Cl2 (146 mg, 0.20 mmol) and 4-(4,4,5,5-tetraacetic acid-1,3,2-dioxborane-2-acyl)pyridine (345 mg, 1.68 mmol) in 1,4-dioxane/H₂O 9.6 ml were stirred at 80°C for 2 hours. The reaction mixture was concentrated under vacuum to obtain a crude product, which was purified by flash chromatography on a 40 g silica gel column with MeOH/DCM (0-10%) gradient elution. In the end, 270 mg of white solid was obtained. Analysis result: Suzuki coupling was successful. LC-MS: RT = 1.30 min; ESI m/z [M+H]⁺ = 297 FA mobile system.

### 8.2 Synthesis of 1-(2,3-dihydrobenzo[b][1,4]dioxin-6-yl)-3-(5-(pyridin-4-yl)isoindolin-2-yl)propan-1-one (41)

To a solution of 5-(pyridine-4-acyl)isoindoline (270 mg, 1.37 mmol), Et₃N (418 mg, 4.14 mmol), DMAP (34 mg, 0.28 mmol) in 15ml of THF was added 3-chloro-1-(2,3-dihydrobenzo[1,4]dioxin-6-acyl)-propyl-1-one (313 mg, 1.38 mmol). Then it was stirred at room temperature for 4 hours. 20 ml of water was added to the reaction mixture, and the mixture was stirred for 10 min, allowed to stand still to separate into layers, and the aqueous layer was extracted once with 20 ml of EA. Then the organic layer was mixed and washed with saturated NaHCO₃ 10 ml×2 and brine 10 ml, the organic solvent was concentrated under vacuum to obtain a crude product, which was purified by flash column chromatography on a 40 g silica gel column with DCM/MeOH gradient elution (0-10%). In the end, 80 mg of yellow oil was obtained. Analysis result: yield 15.1%. LC-MS: RT = 1.01 min; >95% purity; ESI m/z [M+H]⁺ = 387 FA mobile system. ¹H NMR (500MHz, DMSO-*d*₆) δ8.61(d, *J* = 4.0 Hz, 2H), 7.69-7.66 (m, 2H), 7.62 (d, *J=* 7.5 Hz, 1H), 7.55 (d, *J=* 10.5 Hz, 1H), 7.50 (s, 1H), 7.36 (d, *J* = 8.0 Hz, 1H), 6.98 (d, *J=* 8.0 Hz, 1H), 4.33-4.29 (s, 4H), 3.95-3.93 (m, 4H), 3.20 (t, *J=* 7.0 Hz, 2H), 3.06 (t, *J=* 7.0 Hz, 2H).

### Example 9

### Synthesis of 1-(2,3-dihydrobenzo[b][1,4]dioxin-6-yl)prop-2-en-1-one (42)

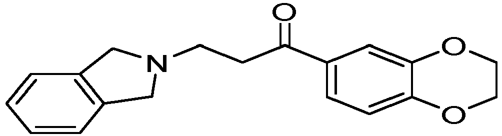

### Route 9. Synthesis of compound 42.

3-bromo-1-(2,3-dihydrobenzo[b][1,4]dioxin-6-acyl)-propyl-1-one (100 mg, 0.369 mmol), DCM 5 ml were mixed in a 20 ml vial. The mixture was cooled to 10. 149 mg, 1.48 mmol of tea leaves were added. The mixture was stirred at room temperature overnight. The reaction was stopped and concentrated under vacuum to obtain a crude product. Flash column chromatography with 25 g silica gel column with MeOH (methanol)/DCM (dichloromethane) (0-5%) gradient elution was used to obtain a white solid (80 mg). Analysis result: yield 57.1%. LC-MS: EPN18027-060-A Rt = 1.45 min; Purity 99.0%; ESI m/z [M+1]⁺= 191.20.

### Example 10 Method of preparing compounds 43, 44.

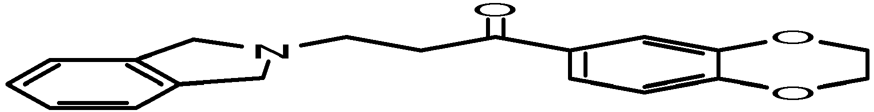

### Route 10. Synthesis of compounds 43, 44.

### 10.1 Synthesis of 1-(2,3-dihydrobenzo[b][1,4]dioxin-6-yl)-3-(3-(4-fluorophenyl)-3-hydroxypyrrolidine-1-yl)propan-1-one (43)

A solution of 1.3 ml (1.3 mmol) of compound 43a was added dropwise to a solution of 17 (297 mg, 1.08 mmol) in 3 ml of THF, and the mixture was placed in a 50 ml flask, and heated at 0°C for 1 hour. The reaction mixture was poured into an aqueous ammonium chloride solution, and extracted with EA 10 ml×3. The organic layers were combined, washed with 5 ml brine, and dried over anhydrous magnesium sulfate for 4 hours. After removing the solvent, flash column chromatography on a 25 g silica gel column with MeOH (methanol)/DCM (dichloromethane) (0-5%) gradient elution was used to obtain a white solid (20 mg).

### 10.2 Synthesis of 1-(2,3-dihydrobenzo[b][1,4]dioxin-6-yl)-3-(3-hydroxy-3-phenylpyrrolidin-1-yl)propan-1-one (44)

1-(3-(2,3-(2,3-dihydrobenzo[b][1,4]dioxin-6-acyl)-3-oxopropyl)pyrrolidone-3- 1,200 mg( 0.728 mmol), 5 ml of THF were mixed in a 20 ml vial, and placed in acetone with dry ice to cool to -40°C. 0.728 ml (0.728 mmol) of benzene magnesium bromide was added dropwise into the mixture, and the mixture was stirred at -40°C for 1 hour. 2 ml of saturated NH₄Cl was added dropwise to stop the reaction. 5 ml of cold water was added to the reaction mixture, and the mixture was stirred for 10 min, allowed to stand still to separate into layers, and then the aqueous layer was extracted with EA 20 ml×2. Then the organic layer was mixed and washed with sat5 ml brine. The organic solvent was concentrated under vacuum to obtain a crude product, which was purified by flash column chromatography on an 80 g silica gel column with MeOH (methanol)/DCM (dichloromethane) (0-5%) gradient elution. In the end, 20 mg of White solid was obtained. Analysis result: yield 7.8%. LC-MS: EPN18027-031-A RT = 1.21 min; 90.5% purity; ESI m/z [M+1]⁺=354.42.

### Example 11

### 11. General preparation method of compounds 45-50.

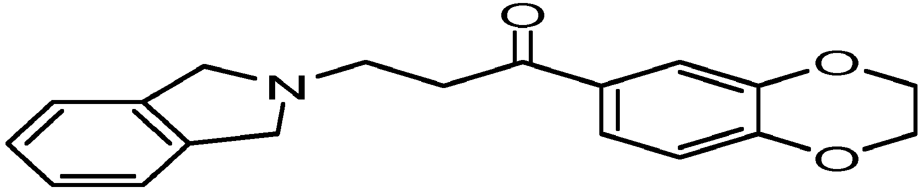

### Route 11. Synthesis of compounds 45-50.

### 11.1.1 Synthesis of tert-butyl 3-hydroxy-3-(4-(trifluoromethyl)phenyl)pyrrolidine-1-carboxylate (45b)

A solution of 45a (6.67 mmol) was added to THF (30 mL), and n-BuLi (2.5 M in hexane, 3.5 mL, 8.67 mmol) was added dropwise at -78°C. After the addition, it was stirred at -78°C for 1 hour. A solution of tert-butyl 3-oxopyrrolidone-1-carboxylate (1.85 g, 9.99 mmol) in THF (5 mL) was added dropwise. The added mixture was stirred at -78°C for 3 hours. Then the mixture was quenched with NH4C1 (sat., 50ml), extracted with EtOAc (50ml 2), and the mixed organic solution was dried over Na₂SO₄ and filtered. After the filtrate was concentrated, the residue was purified by silica gel column chromatography (petroleum ether: EtOAc = 4:1) to obtain the desired product 45b (1.0 g, yield 45%) as a white solid. 1H NMR (400 MHz, CDCl₃): δ 7.66-7.60 (m, 4H), 3.78-3.57 (m, 4H), 2.38-2.14 (m, 2H), 2.13-2.04 (m, 1H), 1.48 (s, 9H).

### 11.1.2 Synthesis of 3-(4-(trifluoromethyl)phenyl)-2,5-dihydro-1H-pyrrole 2,2,2-trifluoroacetate (45c)

To a solution of tert-butyl 3-hydroxy-3-(4-(trifluoromethyl)phenyl)pyrrolidone-1-carboxylate (45b) (700 mg, 2.10 mmol) in TFA (5ml) was added triethyl silane (2ml). The mixture was stirred at 105°C for 12 hours. The mixture was concentrated to obtain the product 45c (1.0 g crude product) as a yellow crude product, which was used directly in the next step. LCMS [Mobile phase: from 95% water (0.02% NH₄OAc) and 5% CH₃CN to 5% water (0.02% NH₄OAc) and 95% CH₃CN, within 3 min], Rt = 1.355 min; MS calculated value: 213; MS measured value: 214 [M+H]⁺.

### 11.1.3 Synthesis of 3-(4-(trifluoromethyl)phenyl)pyrrolidine 2,2,2-trifluoroacetate (45d)

3-(4-(trifluoromethyl)phenyl)-2,5-dihydro-1H-pyrrole 2,2,2-trifluoroacetate (45c) (1.0 g crude product, 2.10 mmol) was added to MeOH (20ml), Pd/C (100mg). The mixture was stirred overnight at 40°C under H2 (50 psi). Then the mixture was filtered, and the filtrate was concentrated to obtain a crude product 45d (691 crude product) as a yellow solid, which was directly used in the next step.

LCMS [Mobile phase: from 95% water (0.02% NH₄OAc) and 5% CH₃CN to 5% water (0.02% NH₄OAc) and 95% CH₃CN in 3 min], Rt = 1.363 min; MS calculated value: 215; MS measured value: 216 [M+H]⁺.

### 11.1 Synthesis of 1-(2,3-dihydrobenzo[b][1,4]dioxin-6-yl)-3-(3-(4-(trifluoromethyl)phenyl)pyrrolidine-1-yl)propan-1-one (45)

3-(4-(trifluoromethyl)phenyl)pyrrolidine 2,2,2-trifluoroacetate (45d) (400 mg, 1.22 mmol) and DIPEA (315 mg, 2.44 mmol) was mixed in DCM (15ml), and 3-bromo-1-(2,3-dihydrobenzo[b][1,4]dioxin-6-yl)-propyl-1-one (i, 397 mg, 1.46 mmol) was added. The resulting mixture was stirred at room temperature for 3 hours. Then the reaction mixture was concentrated, and the residue was purified with a C18 column (CH₃CN/water, 40%-70%, 40 min) to obtain the title compound 45 (50 mg, yield 10%) as a white solid. LC-MS [Mobile phase: from 60% water (0.02% NH₄OAc) and 40% CH₃CN to 30% water (0.02% NH₄OAc) and 70% CH₃CN, within 6.5 min], purity: 98.89% (214 nm), 96.25% (254 nm); Rt = 3.273 min; MS calculated value: 405; MS measured value: 406 [M+H]⁺. ¹H NMR (400 MHz, DMSO-*d*₆): δ 7.61 (d, *J* = 8.0 Hz, 2H), 7.53-7.47 (m, 4H), 6.96 (d, *J* = 8.0 Hz, 1H), 4.33-4.27 (m, 4H), 3.42-3.35 (m, 1H), 3.17-3.07 (m, 2H), 2.91-2.70 (m, 4H), 2.67-2.61 (m, 1H), 2.58-2.51 (m, 1H), 2.32-2.19 (m, 1H), 1.75-1.64 (m, 1H).

### 11.2 Synthesis of 1-(2,3-dihydrobenzo[b][1,4]dioxin-6-yl)-3-(3-(4-nitrophenyl)pyrrolidin-1-yl)propane-1-one hydrochloride (46)

In DCM (2 mL) was added a mixture of 3-(4-nitrophenyl)pyrrolidone hydrochloride (40 mg, 0.17 mmol) and DIEA (88 mg, 0.68 mmol). After stirring for 5 minutes, the mixture was cooled to 5°C. Then in DCM (1ml) was added a solution of 3-bromo-1-(2,3-dihydro-benzo[1,4]dioxin-6-acyl)-propyl-1-one (55 mg, 0.20 mmol ). The resulting mixture was stirred at 5-15°C for 2 hours. The reaction solution was poured into water (10 ml), extracted with DCM (10 ml 3), and the combined organic phase was washed with brine (10 ml), dried over Na₂SO₄ and filtered. After the filtrate was concentrated, the residue was purified by C18 column chromatography (20-50% MeCN aqueous solution, 15 min) to obtain a crude product (45 mg) as a yellow solid. Then it was further purified by C18 [10-50% MeCN in water (0.1% hydrochloride), 10 min] column chromatography to obtain the desired product 46 (35 mg, yield 48%) as a yellow solid.

LC-MS [Mobile phase: from 95% water (0.02% NH₄OAc) and 5% CH₃CN to 5% water (0.02% NH₄OAc) and 95% CH₃CN, within 6.5 min], purity: 95.82% (214 nm), 95.00% (254 nm); Rt = 3.870 min; MS calculated value: 382; MS measured value: 383 [M+H]⁺. ¹H NMR (400 MHz, DMSO-*d*₆) δ 11.43-11.25 (m, 1H), 8.30-8.16 (m, 2H), 7.75-7.68 (m, 2H), 7.56-7.51 (m, 2H), 7.02 (d, *J* = 8.8 Hz, 1H), 4.36-4.29 (m, 4H), 4.02-3.88 (m, 1H), 3.84-3.74 (m, 6H), 3.42-3.37 (m, 1H), 3.30-3.19 (m, 1H), 2.48-2.42 (m, 1H), 2.20-1.98 (m, 1H).

### 11.3 Synthesis of 3-(3-(4-bromophenyl)pyrrolidin-1-yl)-1-(2,3-dihydrobenzo[b][1,4]dioxen-6-yl)propan-1-one (47)

3-(4-bromophenyl)pyrrolidine 2,2,2-trifluoroacetate (49 mg, 0.14 mmol) and 3-bromo-1-(2,3-dihydrobenzo[b][1,4]dioxin-6-yl)-propyl-1-one (45 mg, 0.18 mmol), DIEA (54 mg, 0.42 mmol) were added to DCM (5 mL). The resulting mixture was stirred at room temperature for 2 hours. The reaction mixture was concentrated, and the residue was purified with a C18 column (CH₃CN/water, 45%-75%, 40min) to obtain the title compound 47 (20mg, yield 34%) as a yellow solid.

LC-MS [Mobile phase: from 95% water (0.02% NH₄OAc) and 5% CH₃CN to 5% water (0.02% NH₄OAc) and 95% CH₃CN, within 6.5 min], purity: 96.48% (214 nm), 91.25% (254 nm); Rt = 4.081 min; MS calculated value: 415; MS measured value: 416 [M+H]⁺. 1H NMR (400 MHz, CDCl₃): δ 7.52-7.50 (m, 2H), 7.40-7.38 (m, 2H), 7.13 (d, *J* = 6.8 Hz, 1H), 6.92-6.90 (m, 1H), 4.35-4.26 (m, 4H), 3.38-3.26 (m, 1H), 3.17-3.14 (m, 2H), 3.09-3.06 (m, 2H), 2.97-2.89 (m, 1H), 2.87-2.73 (m, 2H), 2.62-2.53 (m, 1H), 2.38-2.27 (m, 1H), 1.88-1.76 (m, 1H).

### 11.4 Synthesis of 1-(2,3-dihydrobenzo[b][1,4]dioxin-6-yl)-3-(3-(p-tolyl)pyrrolidin-1-yl)propan-1-one hydrochloride (48)

To a mixture of to a mixture of 3-(p-tolyl)pyrrolidine (30 mg, 0.19 mmol) and DIEA (48 mg, 0.37 mmol) in DCM (2 ml) was added 3-bromo-1-(2,3-dihydrobenzo)[b][1,4]dioxin-6-acyl)-propyl-1-one (56 mg, 0.20 mmol). The mixture was stirred at room temperature for 2 hours. The reaction solution was poured into 10 ml of water, extracted with DCM (20 ml2), dried over Na₂SO₄, filtered and concentrated. The residue was dissolved in DMSO (1 ml) and conc. Hydrochloric acid (1) was added. It was purified by C18 [10-40% MeCN in water (0.1% hydrochloride), 10 min] column chromatography, to obtain the desired product 48 (30 mg, yield 41%) as a yellow solid. LC-MS [Mobile phase: from 80% water (0.1% TFA) and 20% CH₃CN to 30% water (0.1% TFA) and 70% CH₃CN, within 6.5 min], purity: 99.36% (214 nm), 98.43% (254 nm); Rt=3.011min; MS calculated value: 351; MS measured value: 352[M+H]⁺. ¹H NMR (400 MHz, DMSO-*d*₆): δ 11.47-11.28 (m, 1H), 7.55-7.51 (m, 2H), 7.30-7.24 (m, 2H), 7.18-7.15 (m, 2H), 7.01 (d, *J=* 8.4 Hz, 1H), 4.35-4.30 (m, 4H), 3.91-3.74 (m, 1H), 3.70-3.52 (m, 6H), 3.39-3.32 (m, 1H), 3.21-3.08 (m, 1H), 2.41-2.35 (m, 1H), 2.28 (m, 3H), 2.13-1.94 (m, 1H).

### 11.5 Synthesis of 3-(3-(4-chlorophenyl)pyrrolidin-1-yl)-1-(2,3-dihydrobenzo[b][1,4]dioxin-6-yl)propan-1-one hydrochloride (49)

3-bromo-1-(2,3-dihydrobenzo[b][1,4]dioxin-6-acyl)-propyl-1-one (107 mg, 0.396 mmol) was added to a mixture of 3-(4-chlorophenyl)pyrrolidine (50 mg, 0.330 mmol) and DIEA (85 mg, 0.660 mmol) in dichloromethane (2 mL). The mixture was stirred at room temperature for 2 hours. The reaction solution was poured into 10 ml of water, extracted with dichloromethane (20 ml 2), dried over Na₂SO₄, and concentrated. It was dissolved with N,N-dimethylformamide (1ml) and conc. Hydrochloride (1 drop) was added. It was purified by C18 [10-40% MeCN in water (0.1% hydrochloride)] column chromatography to obtain the desired product 49 (30 mg, yield 41%) as a yellow solid. LC-MS [Mobile phase: from 95% water (0.1% TFA) and 5% CH₃CN to 5% water (0.1% TFA) and 95% CH₃CN, within 6.5 min], Rt = 3.325 min; Purity: 98.12% (214 nm), 97.14% (254 nm); MS calculated value: 371.1; MS measured value: 371.9 [M+H]⁺. ¹H NMR (400 MHz, CDCl₃): δ 13.10 (s, 0.4H), 12.92 (s, 0.6H), 7.54-7.52 (m, 2H), 7.40-7.38 (m, 1H), 7.33-7.31 (m, 2H), 7.18-7.16 (m, 1H), 6.92-6.90 (m, 1H), 4.32-4.31 (m, 2H), 4.28-3.26 (m, 2H), 3.94-3.61 (m, 7H), 3.40-3.89 (m, 0.4H), 3.19-3.16 (m, 0.6H), 3.00-2.88 (m, 0.4H), 2.86-2.81 (m, 0.6H), 2.63-2.61 (m, 0.4 H), 2.47-2.38 (m, 1H), 2.18-2.11 (m, 0.6H).

### 11.6 Synthesis of 4-(1-(3-(2,3-dihydrobenzo[b][1,4]dioxin-6-yl)-3-oxopropyl)pyrrolidin-3-yl)benzonitrile (50)

To 4-(pyrrolidone-3-yl)benzonitrile 2,2,2-trifluoroacetate (75 mg, 0.26 mmol) and 3-bromo-1-(2,3-dihydrobenzo[b][1,4]dioxin-6-yl)-propyl-1-one (85 mg, 0.31 mmol) in DCM (8 mL) was added DIPEA (101 mg, 0.78 mmol). The resulting mixture was stirred at room temperature for 2 hours. Then the reaction mixture was concentrated, and the residue was purified with a C18 column (CH₃CN/water, 45%-78%, 40 min) to obtain the title compound 50 (20 mg, yield 21%) as a yellow solid. LC-MS [Mobile phase: from 90% water (0.1% TFA) and 10% CH₃CN to 30% water (0.1% TFA) and 70% CH₃CN, within 6.5 min], purity: 99.20% (214 nm), 85.27% (254 nm); Rt = 3.162 min; MS calculated value: 362; MS measured value: 363 [M+H]⁺. ¹H NMR (400 MHz, CDCl₃): δ 7.57-7.50 (m, 4H), 7.36 (d, *J=* 8.0 Hz, 2H), 6.91 (d, *J* = 9.2 Hz, 1H), 4.33-4.28 (m, 4H), 3.45-3.31 (m, 1H), 3.21-3.09 (m, 2H), 3.04-2.91 (m, 3H), 2.89-2.73 (m, 2H), 2.69-2.59 (m, 1H), 2.41-2.28 (m, 1H), 1.89-1.75 (m, 1H).

### Example 12

### 12. General preparation method of compounds 51, 52.

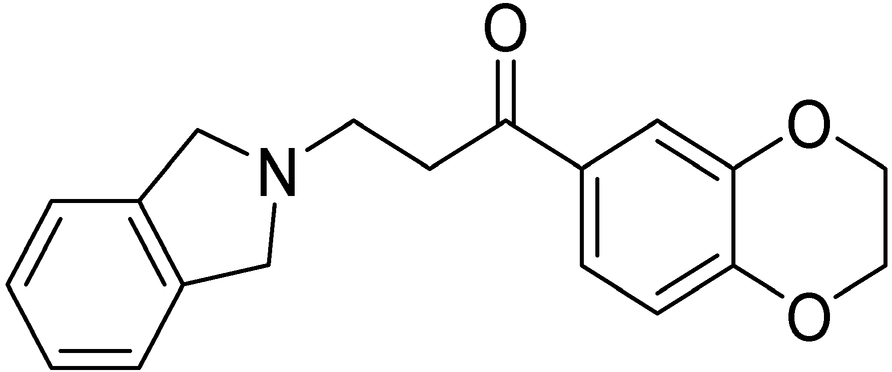

### 12.1.1 Synthesis of 1-bromo-4-fluoro-2-(methoxymethoxy)benzene (51a)

To a solution of 2-bromo-5-fluorophenol (1)(4.50 g, 23.6 mmol) in DCM (50 mL) was added DIEA (6.09 g, 47.2 mmol). Then bromo(methoxy)methane (3.25 g, 26.0 mmol) was added at 0°C. The mixture was stirred at 0°C for 1 hour. The mixture was then washed with water (50 mL) and brine (50 mL), dried over Na₂SO₄ and concentrated. The product was purified by silica gel column chromatography (petroleum ether: EtOAc = 50:1) to obtain the title compound (5.0 g, yield 90%) as a colorless oil. ¹H NMR (400 MHz, CDCl₃) δ 7.47 (dd, *J* = 8.4, 6.4 Hz, 1H), 6.93 (dd, *J* = 10.4, 2.8 Hz, 1H), 6.66-6.62 (m, 1H), 5.23 (s, 2H), 3.52 (s, 3H).

### 12.1.2 Synthesis of 3-(4-fluoro-2-(methoxymethoxy)phenyl)-3-hydroxypyrrolidine-1-carboxylic acid benzyl ester (51b)

In THF (30ml) was added a solution of 1-bromo-4-fluoro-2-(methoxymethoxy)benzene (5.00 g, 21.3 mmol) and N1,N1,N2,N2-tetramethylethane-1,2-diamine (3.71 g, 32.0 mmol). n-BuLi (2.5 M in hexane, 12.8 mL, 32.0 mmol) was added dropwise at -70°C. After the addition, it was stirred at -70°C for 1 hour. Then a solution of 3-oxopyrrolidone-1-carboxylic acid benzyl ester (4.66 g, 21.3 mmol) was added dropwise at - 70°C. After the addition, it was stirred at -70°C for 1 hour. Water (20 mL) was added to cool the reaction. The mixture was then warmed to room temperature, extracted with EtOAc (30 mL 2), and the combined organic phase was washed with brine (30 mL), dried over Na₂SO₄ and concentrated. The residue was purified by silica gel column chromatography (petroleum ether: EtOAc, 10:1 to 5:1) to obtain the title compound 51b (2.4 g, yield 30%) as a yellow oil.

¹H NMR (400 MHz, CDCl₃) δ 7.41-7.31 (m, 5H), 7.23-7.19 (m, 1H), 6.95 (dd, *J* = 17.6, 8.4 Hz, 1H), 6.77-6.72 (m, 1H), 5.30-5.25 (m, 2H), 5.17-5.16 (m, 2H), 4.31-4.18 (m, 1H), 3.74-3.65 (m, 3H), 3.51-3.43 (m, 3H), 2.58-2.37 (m, 2H).

### 12.1.3 Synthesis of 3-(4-fluoro-2-hydroxyphenyl)-2,5-dihydro-1H-pyrrole-1-carboxylic acid benzyl ester (51c)

Benzyl 3-(4-fluoro-2-(methoxymethoxy)phenyl)-3-hydroxypyrrolidone-1-carboxylate (51b) (1.40 g, 3.73 mmol), TsOH (64 mg, 0.37 mmol) were added to toluene (20ml). The mixture was heated to 100°C, stirred for 30 min, and the reaction solution was cooled and concentrated. The residue was purified by silica gel column chromatography (petroleum ether: EtOAc, 10:1-2:1) to obtain the desired product 5 (700 mg, yield 60%) as a white solid.

¹H NMR (400 MHz, DMSO-*d*₆) δ 10.25-10.23 (m, 1H), 7.40-7.32 (m, 5H), 7.16-7.10 (m, 1H), 6.74-6.72 (m, 1H), 6.68-6.63 (m, 1H), 6.26-6.24 (m, 1H), 5.13 (s, 2H), 4.52-4.48 (m, 2H), 4.30-4.25 (m, 2H).

### 12.1.4 Synthesis of 3-(4-fluoro-2-(methoxymethoxy)phenyl)-2,5-dihydro-1H-pyrrole-1-carboxylic acid benzyl ester (51d)

Benzyl 3-(4-fluoro-2-hydroxyphenyl)-2,5-dihydro-1H-pyrrole-1-carboxylate (51c) (700 mg, 2.24 mmol), DIEA (578 mg, 4.48 mmol) were added to DCM (20ml). Then bromomethoxymethane (280 mg, 2.24 mmol) was added at 0°C. After the addition, it was stirred at 0°C for 20 min, then washed with water (15 mL) and brine (20 mL), dried over Na₂SO₄ and concentrated. The residue was purified by silica gel column chromatography (petroleum ether: EtOAc = 10:1) to obtain the title compound 51d (340 mg, yield 43%) as a colorless oil.

¹H NMR (400 MHz, CDCl₃) δ 7.42-7.30 (m, 5H), 7.21-7.15 (m, 1H), 6.93 (d, *J* = 9.2 Hz, 1H), 6.79-6.73 (m, 1H), 6.10 (d, *J=* 21.2 Hz, 1H), 5.20-5.18 (m, 4H), 4.60-4.56 (m, 2H), 4.40-4.38 (m, 2H), 3.47-3.45 (m, 3H).

### 12.1.5 Synthesis of 3-(4-fluoro-2-(methoxymethoxy)phenyl)pyrrolidine (51e)

To CH3OH (5ml) was added a solution of benzyl 3-(4-fluoro-2-(methoxymethoxy)phenyl-2,5-dihydro-1H-pyrrole-1-carboxylate (51d) (240mg), 0.672 mmol), Pd/C (10%, 50mg) and CH₃COOH (4 drops). The mixture was stirred at room temperature under H2 atmosphere (1atm) for 1 hour. Then the mixture was filtered and the filtrate was concentrated. The residue was dissolved in EtOAc ( 10 mL). The mixture was washed with NaHCO₃ solution (sat., 10ml) and brine (10ml), dried over Na₂SO₄ and concentrated. The residue was purified by C18 column chromatography (5-70% MeCN in water, 30min) to obtain the desired product 51e (50 mg, yield 33%) as a yellow oil. ¹H NMR (400 MHz, CDCl₃) δ 7.12-7.06 (m, 1H), 6.87 (d, *J* = 8.4 Hz, 1H), 6.73-6.69 (m, 1H), 5.19 (s, 2H), 3.74-3.68 (m, 1H), 3.48 (s, 3H), 3.24-3.15 (m, 2H), 3.08-2.96 (m, 2H), 2.11-2.04 (m, 1H), 2.00-1.93 (m, 1H).

### 12.1.6 Synthesis of 1-(2,3-dihydrobenzo[b][1,4]dioxin-6-yl)-3-(3-(4-fluoro-2-(methoxymethoxy)phenyl)pyrrolidin-1-yl)propan-1-one (51f)

In DCM (3ml) was added a solution of 3-(4-fluoro-2-(methoxymethoxyphenyl)pyrrolidine (51e) (50mg, 0.22 mmol) and 3-bromo-1-(2,3-dihydrobenzo[b][1,4]dioxin-6-yl)propan-1-one (1ii) (70mg, 0.26 mmol). The mixture was stirred at room temperature for 2 hours. Then the mixture was concentrated, and the residue was purified by C18 [5-70% MeCN in water, 40min] column chromatography to obtain the desired product 51f (60mg, yield 66%) as a yellow oil. ¹H NMR (400 MHz, CDCl₃) δ 7.53-7.50 (m, 2H), 7.12-7.07 (m, 1H), 6.91 (d, *J=* 9.2 Hz, 1H), 6.86 (d, *J=* 8.4 Hz, 1H), 6.73-6.68 (m, 1H), 5.18 (s, 2H), 4.33-4.27 (m, 4H), 4.02-3.93 (m, 1H), 3.48 (s, 3H), 3.18-3.15 (m, 2H), 3.13-3.09 (m, 2H), 3.03-2.93 (m, 2H), 2.66-2.56 (m, 2H), 2.25-2.16 (m, 1H), 2.11-2.06 (m, 1H).

### 12.1 Synthesis of 1-(2,3-dihydrobenzo[b][1,4]dioxin-6-yl)-3-(3-(4-fluoro-2-hydroxyphenyl)pyrrolidine-1-yl)propan-1-one hydrochloride (51)

In a solution of EtOAc (1ml) was added 1-(2,3-dihydrobenzo[b][1,4]dioxin-6-yl)-3-(3-(4-fluoro-2-(methoxyl)phenyl)pyrrolidone-1- 1 (51f) (50mg, 0.12 mmol) hydrochloride/EtOAc (4m, 2ml). The mixture was stirred at room temperature for 2 hours. The mixture was concentrated, and the residue was distilled three times with EtOAc (2 mL) to obtain a crude product (30 mg). The crude product was purified by column chromatography C18 [10-40% MeCN in water (0.1% hydrochloride), 15min] to obtain the desired product 51 (14 mg, yield) 31%) as a white solid. LC-MS [Mobile phase: from 90% water (0.1% TFA) and 10% CH₃CN to 30% water (0.1% TFA) and 70% CH₃CN, within 6.5 min], purity: 99.05 % (214 nm), 97.52% (254 nm); Rt = 3.253 min; MS calculated value: 371; MS measured value: 372 [M+H]⁺. ¹H NMR (400 MHz, DMSO-*d*₆) δ 10.96 (s, 1H), 10.46-10.40 (m, 1H), 7.55-7.52 (m, 2H), 7.15-7.09 (m, 1H), 7.02 (d, *J* = 8.4 Hz, 1H), 6.77-6.75 (m, 1H), 6.65 (t, *J* = 9.6 Hz, 1H), 4.35-4.30 (m, 4H), 4.04-3.86 (m, 1H), 3.79-3.71 (m, 2H), 3.60-3.48 (m, 4H), 3.28-3.25 (m, 2H), 2.33-2.24 (m, 2H).

### 12.2 Synthesis of 1-(2,3-dihydrobenzo[b][1,4]dioxin-6-yl)-3-(3-(4-fluoro-3-hydroxyphenyl)pyrrolidine-1-yl)propan-1-one (52)

In DCM (3ml) was added 3-(4-fluoro-3-(methoxy)phenyl)pyrrolidone (52f) (45 mg, 0.20 mmol) in a solution of 3-bromo-1-(2,3-dihydrobenzo[b][1,4]dioxin-6-acyl)-propyl-1-one (1ii) (81) mg, 0.30 mmol) and DIPEA (52 mg, 0.40 mmol). The mixture was stirred at room temperature for 1 hour. The mixture was concentrated and the residue was dissolved in EtOAc (15ml). The solution was washed with water (10ml) and brine (10ml), dried over Na₂SO₄ and concentrated. The residue was dissolved in EtOAc (1ml), and hydrochloride/EtOAc (4m, 2ml) was added. It was stirred at room temperature for 30 min. After concentration, the residue was triturated with EtOAc (3 ml). The mixture was filtered. The solid was collected and further purified by C18 [10-40% MeCN, 0.1% hydrochloride in water, 15 min] column chromatography to obtain the desired product 52 (16 mg, yield 20%) as a white solid. LC-MS [Mobile phase: from 80% water (0.1% TFA) and 20% CH₃CN to 30% water (0.1% TFA) and 70% CH₃CN, within 6.5 min], purity: 92.79% (214 nm), 93.01% (254 nm); Rt = 2.522 min; MS calculated value: 371; MS measured value: 372 [M+H]⁺. ¹H NMR (400 MHz, DMSO-*d*₆): δ 10.96-10.81 (m, 1H), 9.92 (s, 1H), 7.55-7.51 (m, 2H), 7.15-7.09 (m, 1H), 7.02 (d, *J=* 8.4 Hz, 1H), 6.95-6.92 (m, 1H), 6.82-6.77 (m, 1H), 4.35-4.29 (m, 4H), 3.91-3.73 (m, 1H), 3.67-3.51 (m, 6H), 3.39-3.33 (m, 1H), 3.20-3.05 (m, 1H), 2.41-2.31 (m, 1H), 2.10-1.88 (m, 1H).

### Example 13

### 13.General preparation method of compounds 53,54.

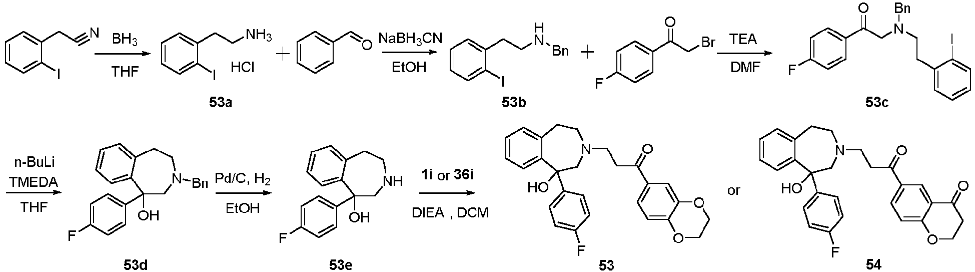

### Route 13. Synthesis of compounds 53,54.

### 13.1.1 Synthesis of 2-(2-iodophenyl)ethylamine hydrochloride (53a)

A solution of 2-(2-Iodophenyl)acetonitrile (4.00 g, 16.5 mmol) was added to THF (12ml), and borane/THF (1m, 40ml, 40mmol) was added dropwise at room temperature. The mixture was then refluxed for 10 hours. The mixture was cooled to 0°C and 10ml ethanol and hydrochloride/ethanol (1m, 20ml, 20mmol) were added. The resulting suspension was concentrated to a crude product and dispersed in acetone (20 mL). Then the mixture was filtered and the solid was collected to obtain the title compound 53a (3.1 g crude product) as a white solid, which was used in the next step without further purification. LCMS [Mobile phase: from 95% water (0.02% NH₄OAc) and 5% CH₃CN to 5% water (0.02% NH₄OAc) and 95% CH₃CN within 2.5 min], Rt = 1.25 min; MS calculated value: 247; MS measured value: 248 [M+H]⁺.

### 13.1.2 Synthesis of N-benzyl-2-(2-iodophenyl)ethylamine (53b)

2-(2-iodophenyl)ethylamine hydrochloride (1.00 g, 3.53 mmol) and benzaldehyde (374 mg, 3.53 mmol) were stirred in ethanol (18 mL) at room temperature for 1 hour. Then sodium cyanide (556 mg, 8.83 mmol) was added, and the mixture was stirred at room temperature overnight. It was mixed with water (30 ml) to be quenched, and extracted with ethyl acetate (2) 30 ml. The combined organic layers were concentrated and purified by column chromatography (acetonitrile: water (0.1% ammonium bicarbonate) from 65% to 70%) to give the title compound 53 b (560 mg, yield 51%) as a white oil.

LC-MS [Mobile phase: from 95% water (0.02% NH₄OAc) and 5% CH₃CN to 5% water (0.02% NH₄OAc) and 95% CH₃CN within 2.5 min], Rt = 1.78 min; MS calculated value: 337; MS measured value: 338 [M+H]⁺.

### 13.1.3 Synthesis of 2-(benzyl(2-iodophenylethyl)amino)-1-(4-fluorophenyl)ethanone (53c)

A solution ofN-benzyl-2-(2-iodophenyl)ethanolamine (53b) (460 mg, 1.37 mmol) and triethylamine (145 mg, 1.43 mmol) was added to DMF (7ml), and 2-bromo-1-(4-fluorobenzene)ethanol (309 mg, 1.43 mmol) was added at 0°C. The mixture was stirred at room temperature for 5 hours. Then water (30 mL) and ethyl acetate (30 mL×2) were added to extract the desired compound. The organic layer was concentrated and purified by column chromatography on C18 (acetonitrile: 95%-100% water) to obtain the title compound 53c (542 mg, yield 84%) as a white oil. ¹H NMR (400 MHz, CDCl₃): δ 7.95-7.90 (m, 2H), 7.75 (d, *J* = 9.2 Hz, 1H), 7.33-7.26 (m, 4.5H), 7.25-7.18 (m, 1.5H), 7.14-7.11 (m, 1H), 7.07-7.02 (m, 2H), 6.87-6.83 (m, 1H), 3.90 (s, 2H), 3.84 (s, 2H), 2.98-2.93 (m, 2H), 2.89-2.84 (m, 2H).

### 13.1.4 Synthesis of 3-benzyl-1-(4-fluorophenyl)-2,3,4,5-tetrahydro-1H-benzo[d]azetidine-1-ol (53d)

A solution of N1,N1,N2,N2-tetramethyl-1,2-diamine (240 mg, 2.07 mmol) and n-butyllithium (2.5 M toluene, 0.83 mL, 2.07 mmol) was added to a solution of tetrahydrofuran (10ml), and a solution of 2-(benzyl(2-iodophenethyl)-1-(4-fluorophenethyl)ethanol (53c) (560 mg, 1.18 mmol) THF (13 mL) was added dropwise under nitrogen atmosphere at -78°C The added mixture was stirred overnight at room temperature. The mixture was quenched with water (10ml), extracted with ethyl acetate (15ml 2), and purified by column chromatography on C18 (acetonitrile: 60% ~ 65% water) to obtain the title compound 53d (100mg, yield 24%) as a yellow oil. LCMS [Mobile phase: from 95% water (0.02% NH₄OAc) and 5% CH₃CN to 5% water (0.02% NH₄OAc) and 95% CH₃CN, 2.5 within min], Rt = 1.93 min; MS calculated value: 347; MS measured value: 348 [M+H]⁺.

### 13.1.5 Synthesis of 1-(4-fluorophenyl)-2,3,4,5-tetrahydro-1H-benzo[d]aza-1-ol (53e)

3-benzyl-1-(4-fluorophenyl)-2,3,4,5-tetrahydro-1H-benzo(d)azopine-1-ol (53d) (100mg, 0.288 mmol) was added to ethanol (15ml) Pd/C (10%, 50mg) at room temperature. The mixture was then stirred under a hydrogen atmosphere (50 psi) at 50°C overnight. The reaction mixture was filtered, and the filtrate was concentrated under reduced pressure to obtain the title compound 53e (65 mg, yield 88%) as a white solid. LCMS [Mobile phase: from 95% water (0.02% NH₄OAc) and 5% CH₃CN to 5% water (0.02% NH₄OAc) and 95% CH₃CN within 2.5 min], Rt = 1.47 min; MS calculated value: 257; MS measured value: 258 [M+H]⁺.

### 13.1 Synthesis of 1-(2,3-dihydrobenzo[b][1,4]dioxin-6-yl)-3-(1-(4-fluorophenyl)-1-hydroxy-4,5-dihydro-1H-benzo[]d]aza-3(2H)-yl)propan-1-one (53)

3-bromo-1-(2,3-dihydrobenzo[b][1,4]dioxin-6-acyl)-propyl-1-one (1i, 102 mg, 0.379 mmol) was added to a solution of 1-(4-fluorophenyl)-2,3,4,5-tetrahydro-1H-benzo[d]azopine-1-ol (53e) (65 mg, 0.25 mmol) and DIPEA (65 mg, 0.51 mmol) in dichloromethane (3ml) at room temperature. The mixture was stirred at room temperature for 2 hours. Then water (10ml) and dichloromethane (10ml) were added to extract the desired compound. The organic layer was concentrated and purified by column chromatography on C18 (acetonitrile: 30%-65% water) to obtain the title compound 53 (20.7 mg, yield 18%) as a white solid. LC-MS [Mobile phase: from 98% water (0.1% TFA) and 2% CH₃CN to 40% water (0.1% TFA) and 60% CH₃CN, within 6.5 min], purity: 92.27% (214 nm), 93.90% (254 nm); Rt = 4.398 min; MS calculated value: 447; MS measured value: 448 [M+H]⁺. ¹H NMR (400 MHz, DMSO-*d*₆): δ 7.52-7.49 (m, 2H), 7.45 (d, *J=* 2.0 Hz, 1H), 7.24-7.14 (m, 4H), 7.09-7.07 (m, 1H), 7.01-6.95 (m, 3H), 5.61 (s, 1H), 4.33-4.28 (m, 4H), 3.47-3.44 (m, 1H), 3.10 (t, *J* = 6.8 Hz, 2H), 2.90-2.84 (m, 3H), 2.71-2.61 (m, 3H), 2.47-2.41 (m, 1H).

### 13.2 Synthesis of 6-(3-(1-(4-fluorophenyl)-1-hydroxy-1,2,4,5-tetrahydro-3H-benzo[d] azazepine-3-yl)propionyl)chroman-4-one(54)

1-(4-fluorophenyl)-2,3,4,5-tetrahydro-1H-benzo(d)azopine-1-ol (65 mg, 0.253 mmol) and N,N-ciisopropylethylamine (65 mg, 0.506 mmol) were added to 1-(4-fluorophenyl)-2,3,4,5-tetrahydro-1H-benzo (102 mg, 0.379 mmol) at room temperature, and the mixture was stirred at room temperature for 2 hours. It was then quenched with water (10ml) and extracted with dichloromethane (10ml). The crude product was purified by flash column chromatography on a 40 g silica gel column with EA/PE gradient elution (40-60%). 12 mg of white solid was obtained. Analysis results: 10.3% yield. LC-MS: RT = 1.37 min; Purity: 93.0%; ESI m/z [M+1]⁺ =460.37 ¹H NMR (400 MHz, CDCl₃): δ 8.45-8.44 (m, 1H), 8.09-8.06 (m, 1H),7.34-7.31 (m, 2H), 7.10-6.08 (m, 7H), 4.60 (t,J=6.4Hz, 2H), 2.84 (t,J=6.4Hz, 2H), 1.58 (s, 4H), 1.40-1.24(m, 6H).

**The compounds prepared in the Examples and their structures and names are shown in Table A below.**

**Table A**

| **Compound No.** | **Structure** | **Name** |
|---|---|---|
| 1 | 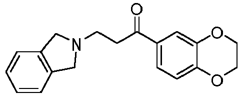 | 1-(2,3-dihydrobenzo[b][1,4]dioxin-6-yl)-3-(isoindolin-2-yl)propan-1-one |
| 2 | 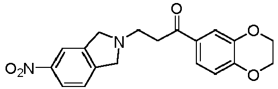 | 1-(2,3-dihydrobenzo[b][1,4]dioxin-6-yl)-3-(5-nitroisoindol-2-yl)propan-1-one |
| 3 | 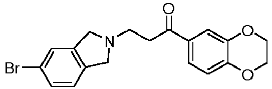 | 3-(5-bromoisoindolin-2-yl)-1-(2,3-dihydrobenzo[b][1,4]dioxin-6-yl)propan-1-one |
| 4 | 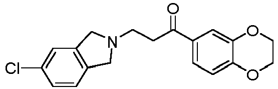 | 3-(5-chloroisoindolin-2-yl)-1-(2,3-dihydrobenzo[b][1,4]dioxin-6-yl)propan-1-one |
| 5 | 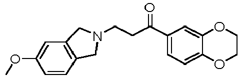 | 1-(2,3-dihydrobenzo[b][1,4]dioxin-6-yl)-3-(5-methoxyisoindolin-2-yl)propan-1-one |
| 6 | 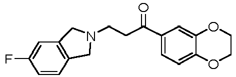 | 1-(2,3-dihydrobenzo[b][1,4]dioxin-6-yl)-3-(5-fluoroiso-2-yl)propan-1-one |
| 7 | 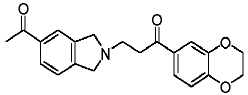 | 3-(5-acetylisoindolin-2-yl)-1-(2,3-dihydrobenzo[b][1,4]dioxin-6-yl)propan-1-one |
| 8 | 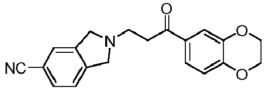 | 2-(3-(2,3-dihydrobenzo[b][1,4]dioxin-6-yl)-3-oxopropyl)isoindole-5-carbonitrile |
| 9 | 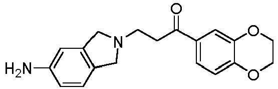 | 3-(5-aminoisoindolin-2-yl)-1-(2,3-dihydrobenzo[b][1,4]dioxin-6-yl)propan-1-one |
| 10 | 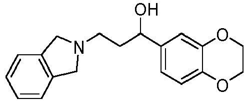 | 1-(2,3-dihydrobenzo[b][1,4]dioxin-6-yl)-3-(isoindolin-2-yl)propan-1-ol |
| 11 | 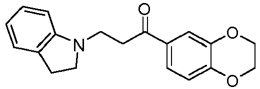 | 1-(2,3-dihydrobenzo[b][1,4]dioxin-6-yl)-3-(indoline-1-yl)propan-1-one |
| 12 | 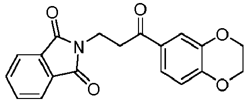 | 2-(3-(2,3-dihydrobenzo[b][1,4]dioxin-6-yl)-3-oxopropyl)isoindole-1,3-dione |
| 13 | 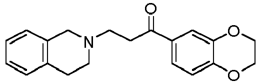 | 1-(2,3-dihydrobenzo[b][1,4]dioxin-6-yl)-3-(3,4-dihydroisoquinoline-2(1H)-yl)propan-1-one |
| 14 | 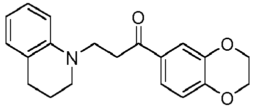 | 1-(2,3-dihydrobenzo[b][1,4]dioxin-6-yl)-3-(3,4-dihydrouinoline-1(2H)-yl)propan-1-one |
| 15 | 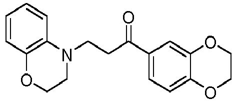 | 3-(2,3-dihydro-4H-benzo[b][1,4]oxazin-4-yl)-1-(2,3-dihydrobenzo[b][1,4]dioxin-6-yl)propan-1-one |
| 16 | 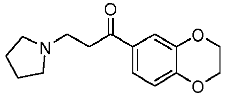 | 1-(2,3-dihydrobenzo[b][1,4]dioxin-6-yl)-3-(pyrrolidin-1-yl)propan-1-one |
| 17 | 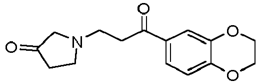 | 1-(3-(2,3-dihydrobenzo[b][1,4]dioxin-6-yl)-3-oxopropyl)pyrrolidin-3-one |
| 18 | 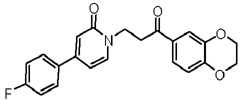 | 1-(3-(2,3-dihydrobenzo[b][1,4]dioxin-6-yl)-3-oxopropyl)-4-(4-fluorophenyl)pyridine-2(1H)-one |
| 19 | 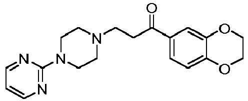 | 1-(2,3-dihydrobenzo[b][1,4]dioxin-6-yl)-3-(4-(pyrimidin-2-yl)piperazin-1-yl)propan-1-one |
| 20 | 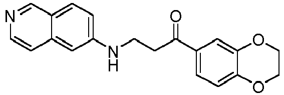 | 1-(2,3-dihydrobenzo[b][1,4]dioxin-6-yl)-3-(isoquinolin-6-ylamino)propan-1-one |
| 21 | 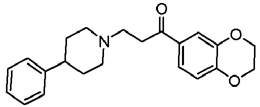 | 1-(2,3-dihydrobenzo[b][1,4]dioxin-6-yl)-3-(4-phenylpiperidin-1-yl)propan-1-one |
| 22 | 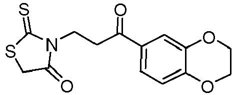 | 3-(3-(2,3-dihydrobenzo[b][1,4]dioxin-6-yl)-3-oxopropyl)-2-thiothiazolidine-4-one |
| 23 | 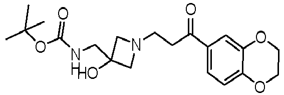 | tert-butyl ((1-(3-(2,3-dihydrobenzo[b][1,4] dioxin-6-yl)-3-oxopropyl)-3-hydroxyazetidin-3-yl)methyl)carbamate |
| 24 | 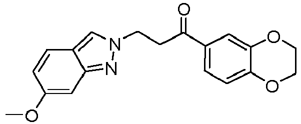 | 1-(2,3-dihydrobenzo[b][1,4]dioxin-6-yl)-3-(6-methoxy-2H-indazol-2-yl)propan-1-one |
| 25 | 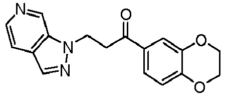 | 1-(2,3-dihydrobenzo[b][1,4]dioxin-6-yl)-3-(1H-pyrazolo[3,4-c]pyridine-1-yl)propan-1-one |
| 26 | 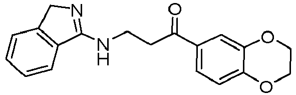 | 3-((1H-isoindol-3-yl)amino)-1-(2,3-dihydrobenzo[b][1,4]dioxin-6-yl)propan-1-one |
| 27 | 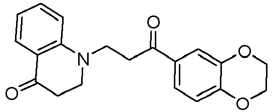 | 1-(3-(2,3-dihydrobenzo[b][1,4]dioxin-6-yl)-3-oxopropyl)-2,3-dihydroquinoline-4(1H)-one |
| 28 | 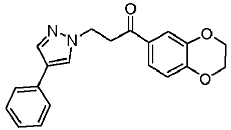 | 1-(2,3-dihydrobenzo[b][1,4]dioxin-6-yl)-3-(4-phenyl-1H-pyrazol-1-yl)propan-1-one |
| 29 | 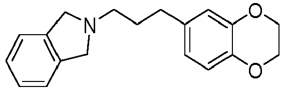 | 2-(3-(2,3-dihydrobenzo[b][1,4]dioxin-6-yl) propyl)isoindoline |
| 30 | 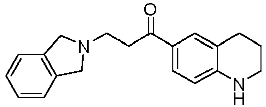 | 3-(isoindolin-2-yl)-1-(1,2,3,4-tetrahydroquinolin-6-yl)propan-1-one |
| 31 | 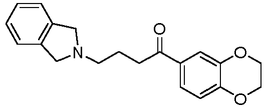 | 1-(2,3-dihydrobenzo[b][1,4]dioxin-6-yl)-4-(isoindolin-2-yl)butan-1-one |
| 32 | 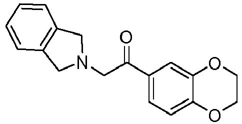 | 1-(2,3-dihydrobenzo[b][1,4]dioxin-6-yl)-2-(isoindolin-2-yl)ethan-1-one |
| 33 | 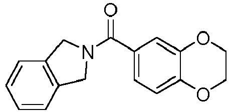 | (2,3-dihydrobenzo[b][1,4]dioxin-6-yl)(isoindolin-2-yl)methanone |
| 34 | 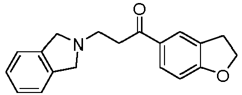 | 1-(2,3-dihydrobenzofuran-5-yl)-3-(isoindolin-2-yl)propan-1-one |
| 35 | 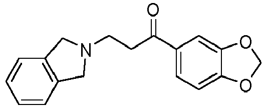 | 1-(benzo[d][1,3]dioxol-5-yl)-3-(isoindolin-2-yl)propan-1-one |
| 36 | 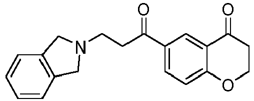 | 6-(3-(isoindolin-2-yl)propionyl)chroman-4-one |
| 37 | 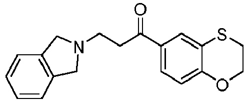 | 1-(2,3-dihydrobenzo[b][1,4]oxathion-6-yl)-3-(isoindolin-2-yl)propan-1-one |
| 38 | 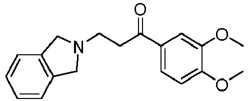 | 1-(3,4-dimethoxyphenyl)-3-(isoindolin-2-yl)propan-1-one |
| 39 | 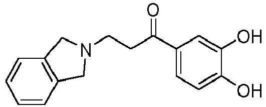 | 1-(3,4-dihydroxyphenyl)-3-(isoindolin-2-yl)propan-1-one |
| 40 | 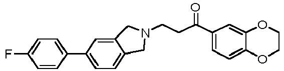 | 1-(2,3-dihydrobenzo[b][1,4]dioxin-6-yl)-3-(5-(4-fluorophenyl)isoindolin-2-yl)propan-1-one |
| 41 | 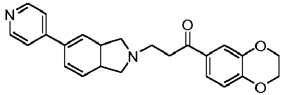 | 1-(2,3-dihydrobenzo[b][1,4]dioxin-6-yl)-3-(5-(pyridin-4-yl)-1,3,3a,7αtetrahydro-2H-isoindolepyridin -2-yl)propan-1-one |
| 42 | 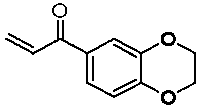 | 1-(2,3-dihydrobenzo[b][1,4]dioxin-6-yl)prop-2-en-1-one |
| 43 | 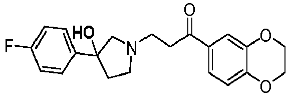 | 1-(2,3-dihydrobenzo[b][1,4]dioxin-6-yl)-3-(3-(4-fluorophenyl)-3-hydroxypyrrolidine-1-yl)propan-1-one |
| 44 | 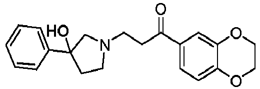 | 1-(2,3-dihydrobenzo[b][1,4]dioxin-6-yl)-3-(3-hydroxy-3-phenylpyrrolidin-1-yl)propan-1-one |
| 45 | 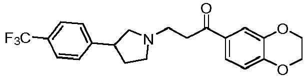 | 1-(2,3-dihydrobenzo[b][1,4]dioxin-6-yl)-3-(3-(4-(tri fluoromethyl)phenyl)pyrroli dine-1-yl)propan-1-one |
| 46 | 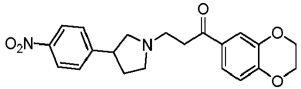 | 1-(2,3-dihydrobenzo[b][1,4]dioxin-6-yl)-3-(3-(4-nitrophenyl)pyrrolidin-1-yl)propan-1-one |
| 47 | 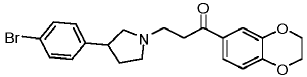 | 3-(3-(4-bromophenyl)pyrrolidin-1-yl)-1-(2,3-dihydrobenzo[b][1,4]dioxin-6-yl)propan-1-one |
| 48 | 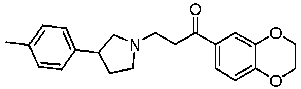 | 1-(2,3-dihydrobenzo[b][1,4]dioxin-6-yl)-3-(3-(p-tolyl)pyrrolidin-1-yl)propan-1-one |
| 49 | 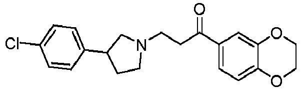 | 3-(3-(4-chlorophenyl)pyrrolidin-1-yl)-1-(2,3-dihydrobenzo[b][1,4]dioxin-6-yl)propan-1-one |
| 50 | 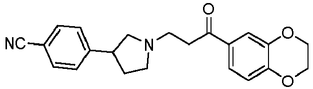 | 4-(1-(3-(2,3-dihydrobenzo[b][1,4]dioxin-6-yl)-3-oxopropyl)pyrrolidin-3-yl)benzonitrile |
| 51 | 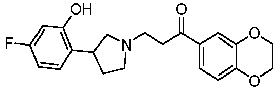 | 1-(2,3-dihydrobenzo[b][1,4]dioxin-6-yl)-3-(3-(4-fluoro-2-hydroxyphenyl)pyrrolidine-1-yl)propan-1-one |
| 52 | 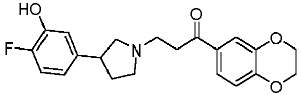 | 1-(2,3-dihydrobenzo[b][1,4]dioxin-6-yl)-3-(3-(4-fluoro-3-hydroxyphenyl)pyrrolidine-1-yl)propan-1-one |
| 53 | 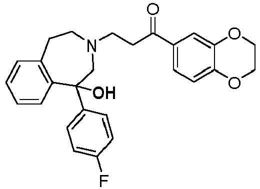 | 1-(2,3-dihydrobenzo[b][1,4]dioxin-6-yl)-3-(1-(4-fluorophenyl)-1-hydroxy- 1 ,2,4,5-tetrahydro-3H-benzo[d]azepine-3-yl)propan-1-one |
| 54 | 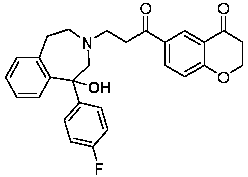 | 6-(3-(1-(4-fluorophenyl)-1-hydroxy-1,2,4,5-tetrahydro-3H-benzo[d]azepine-3-yl)propionyl)chroman-4-one |

### Example 14

### 14.1 Experimental determination of compound's inhibitory rate on TDG mismatch repair activity

(1) A double-stranded DNA probe T1-T2 containing uracil bases was synthesized. The nucleotide sequence of the T1 chain was: 5'-FAM- TAA UGT GAA TGG AGC TGA AAT - biotin-3'; The nucleotide sequence of the T2 chain was 5'-ATT TCA GCT CCA TTC ACG TTA-3'. Using PCR annealing program, the T1 strand and T2 strand were complementary to form a double-stranded DNA probe T1-T2.

(2) The reaction system and steps for each well of a 96-well plate were as follows: a. TDG (100nM/L) was mixed with different concentrations of compounds, and the mixture was incubated at 37°C for 30 minutes. The composition of the reaction buffer was: 20mM HEPES pH7.5, 100mM NaCl, 0.2mM EDTA, 2.5mM MgCl₂; b. The double-stranded DNA probe T1-T2 was added to the mixture to obtain a final volume of 100µL, with a concentration of 30nM, and the mixture was incubated at 25°C for 30min; c. 1µL of streptavidin magnetic beads were added to the reaction solution and fully bound the biotin-labeled DNA for 1 hour; d. NaOH was added to the reaction solution to a final concentration of 200mM, and the mixture was incubated at room temperature for 30 minutes; e. Under the action of a magnetic stand the streptavidin magnetic beads were adsorbed to the tube wall, the supernatant was transferred to a new 96-well plate, and placed in a Tecan microplate reader for fluorescence measurement. The parameter settings were: excitation wavelength 485nm; emission wavelength 520nm. A linear curve between fluorescence intensity and TDG concentration was constructed, R²=0.9968, and the linear range was 0-50nM/L;

According to the biological method described in this example, the selected compounds of the present invention were analyzed, and the results are shown in Table 1. Among them, "++" in Table 1 refers to the inhibitory activity for TDG mismatch repair with IC₅₀≤5 µM; "+" refers to the inhibitory activity for TDG mismatch repair with IC₅₀>5 µM.

### 14.2 Experimental determination of compound's inhibitory rate on the growth of Calu-1 cells

Lung cancer cell line Calu-1 was cultured in 90% McCoy's 5A medium (BI, 01-075-1ACS) + 10% fetal bovine serum (Gibco, 10270106), placed in a CO2 incubator (37°C, 5% CO2, 95% air) for culturing. Calu-1 cells were digested with trypsin (Gibco, 12604-021) and seeded in a 384-well plate at a density of 500 cells per well. The medium was 50 µL and it was cultured overnight at 37°C in an incubator. On the next day, different concentrations of test compounds were added, and the final concentration of DMSO was 0.1%. After 72 hours of culturing, the cells were taken out for test. The cell culture medium from the 384-well plate to be tested was removed, and fresh culture medium premixed with CTG was immediately added: the ratio of the culture medium to CellTiter-Glo^{®} 2.0 reagent (Promega, G9243) was 2:1, 30ul was added to each well. The plate was shaken at room temperature for 20 minutes, and then a multi-function plate reader (TECAN, Infinite 200 Pro) was used according to the reagent instructions to determine the cell survival.

According to the biological method described in this example, the selected compounds of the present invention were analyzed, and the results are shown in Table 1. Among them, "++" in Table 1 refers to the inhibitory activity on the growth of calu-1 cells with IC₅₀≤5 µM; "+" refers to the inhibitory activity on the growth of calu-1 cells with IC₅₀>5 µM.

**Table 1 Results of the inhibitory activity experiments of the compounds on TDG mismatch repair and calu-1 cell growth**

| Compound No. | Inhibitory activity on TDG mismatch repair IC₅₀ (µM) | Inhibitory activity on alu-1 cell growth IC₅₀ (µM) | Compound No. | Inhibitory activity on TDG mismatch repair IC₅₀ (µM) | Inhibitory activity on alu-1 cell growth IC₅₀ (µM) |
|---|---|---|---|---|---|
| 1 | ++ | ++ | 28 | + | + |
| 2 | ++ | ++ | 29 | + | + |
| 3 | ++ | + | 30 | + | + |
| 4 | ++ | + | 31 | + | + |
| 5 | ++ | ++ | 32 | + | + |
| 6 | ++ | + | 33 | + | + |
| 7 | ++ | + | 34 | + | + |
| 8 | ++ | + | 35 | ++ | ++ |
| 9 | ++ | ++ | 36 | ++ | ++ |
| 10 | + | + | 37 | + | + |
| 11 | + | + | 38 | + | + |
| 12 | + | + | 39 | + | + |
| 13 | ++ | ++ | 40 | + | + |
| 14 | + | + | 41 | + | + |
| 15 | + | + | 42 | ++ | ++ |
| 16 | ++ | + | 43 | ++ | ++ |
| 17 | ++ | + | 44 | + | ++ |
| 18 | ++ | + | 45 | + | ++ |
| 19 | + | ++ | 46 | ++ | ++ |
| 20 | + | + | 47 | ++ | ++ |
| 21 | ++ | + | 48 | ++ | ++ |
| 22 | + | + | 49 | ++ | ++ |
| 23 | + | + | 50 | ++ | ++ |
| 24 | + | + | 51 | + | + |
| 25 | + | + | 52 | ++ | ++ |
| 26 | + | + | 53 | ++ | ++ |
| 27 | + | + | 54 | ++ | ++ |

### Example 15

### Experimental determination of TDG inhibitory activity of compounds in cells

The operation steps of this example are as follows:
(1) Preparation of methylation reporter plasmid:
(a) SAM with a stock solution concentration of 32 mM was diluted with ultrapure water to 1600 µM;
(b) The reaction system was prepared according to the following ratio: 30 µl ultrapure water, 5 µl 10X NEBuffer 2, 5 µl diluted SAM, 1 µg pCpGL-CMV-firefly plasmid, 1 µl SssI methylase (4 U/µl) ; 50 µl in total, the reaction was carried out at 37°C for 2 hours;
(c) The mixture was heated at 65°C for 20 minutes. Then 0.2% SDS was added, the reaction was carried out at 80°C for 10min; and then 1µl Proteinase K was added, the reaction was carried out at 50°C for 1h;
(d) The methylated plasmid was recovered with Dr. GenTLE^{®} sedimentation aid (TAKARA).

(2) Preparation of 5fC/5caC pCpGL-CMV-firefly reporter plasmid
(a) The following 10× reaction system was prepared:

| Composition | Working concentration (□M) | Stock solution concentration (M) | Volume (20 µl) |
|---|---|---|---|
| HEPES,pH 8.0^{∗} | 500 | 1 | 10 |
| NaCl | 500 | 5 | 2 |
| Vitamin C | 10 | 0.2 | 1 |
| ATP | 10 | 0.2 | 1 |
| 2-OG | 10 | 0.2 | 1 |
| DTT | 10 | 0.5 | 0.4 |
| (NH₄)₂Fe(SO₄)₂^{∗∗} | 1 | 0.1 | 0.2 |
| ddH₂O | | | 4.4 |

(b) The reaction system was prepared according to the following ratio:

| Component | | Volume (per 50 µl system) | |
|---|---|---|---|
| Tet1 protein | | 24µg | |
| 10 ×reaction system | | 5 µl | |
| Plasmid from step (1) | | 1 µg | |
| ddH₂O | | 50 µl | |

(c) The reaction was carried out at 37°C for one hour;
(d) 24µg Tet1 protein was added for the second time and the reaction was continued for one hour;
(e) SDS was added to a final concentration of 0.5%, 80°C, 10 minutes, then 1µl Proteinase K was added, 55°C overnight.
(f) The treated caC-cmv-firefly plasmid was recovered with Dr. GenTLE^{®} sedimentation aid (TAKARA) and was quantified.
(3) Using the luciferase activity assay method to detect the inhibitory rate of small molecule compounds on intracellular TDG enzyme activity

In a 96-well plate, the reaction system and operation flow of each well were as follows:
(a) 1ng caC-cmv-firefly, 1ng pCpGL-CMV-Relina and 150ng pCDNA4-TDG or 150ng pCDNA4-myc plasmid, were diluted to 75 ul Opti-MEM, mixed well and allowed to stand still for 5 minutes;
(b) 2 ul transfection reagent (Hieff TransTM Liposomal transfection Reagent (0. 5 ul/well, YEAEEN, Cat#40802)) was diluted in 100 ul Opti-MEM, mixed well and allowed to stand still for 5 minutes;
(c) 75 ul of (a) and (b) were mixed respectively, and allowed to stand still at room temperature for 20 minutes;
(d) HEK 293T TDG KO cells were digested with trypsin, the cells were collected and counted, and the suspension cell concentration was adjusted to 4× 10⁵/ml;
(e) 50 ul of the mixture (c) was added to each well, and at the same time 100 ul of the diluted cell suspension was added to the same well, and they were mixed well.
(f) After the cells adhering to the wall, different concentrations of small molecule compounds to be tested were added to each well. The final concentration of DMSO did not exceed 0.1%.
(g) Incubatiton was performed in a 37°C CO2 incubator for 72 hours. The supernatant was removed and the cells were quickly freezed at -80°C.
(h) Before detecting the luciferase activity, the cells to be tested were removed from - 80°C and allowed to stand still at room temperature for 30 minutes. Following the instructions of Dual-Glo^{®} Luciferase Reagent (PROMEGA), the firefly fluorescence value and Renilla fluorescence value of each well were detected.
(i) The firefly fluorescence value was divided with the Renilla fluorescence value of each well; 96-well plate Z' and experimental window was calculated by the ratio of transfected caC-cmv-firefly, pCpGL-CMV-Relina, pCDNA4-TDG plasmid mixture plus DMSO experimental group to transfected caC-cmv-firefly, pCpGL-CMV-Relina, pCDNA4-myc plasmid mixture plus DMSO experimental group, and the relative enzyme activity inhibition rate of each compound was calculated.

According to the biological method described in this example, the selected compounds of the present invention were analyzed, and the results are shown in Table 2. Among them, "+" in Table 2 refers to the compound's TDG inhibitory activity at a concentration of 10 µM ≤**25%**; "++" refers to the compound's TDG inhibitory activity at a concentration of 10 µM >25%.

**Table 2 The results of the compound's TDG inhibitory activity test in cells**

| Compound | Inhibition rate on TDG activity | Compound | Inhibition rate on TDG activity | Compound | Inhibition rate on TDG activity |
|---|---|---|---|---|---|
| **1** | ++ | **19** | + | **37** | + |
| **2** | + | **20** | + | **38** | ++ |
| **3** | + | **21** | + | **39** | + |
| **4** | + | **22** | + | **40** | + |
| **5** | + | **23** | + | **41** | + |
| **6** | + | **24** | + | **42** | + |
| **7** | + | **25** | + | **43** | + |
| **8** | + | **26** | + | **44** | + |
| **9** | ++ | **27** | + | **45** | + |
| **10** | + | **28** | + | **46** | + |
| **11** | + | **29** | ++ | **47** | + |
| **12** | + | **30** | + | **49** | + |
| **13** | + | **31** | + | **50** | ++ |
| **14** | + | **32** | + | **51** | + |
| **15** | + | **33** | + | **52** | + |
| **16** | + | **34** | + | **53** | + |
| **17** | + | **35** | + | **54** | + |
| **18** | ++ | **36** | + | | |

All documents mentioned in the present invention are cited as references in the present application, as if each document was individually cited as a reference. In addition, it should be understood that after reading the above teaching content of the present invention, those skilled in the art can make various changes or modifications to the present invention, and these equivalent forms also fall within the scope defined by the appended claims of the present application.

## Claims

1. A compound represented by formula I, 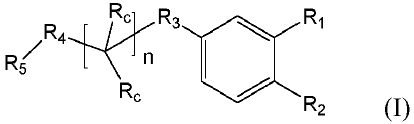 or a stereoisomer or a tautomer, or a pharmaceutically acceptable salt, or a hydrate, or a crystal form, or a solvate thereof,
wherein,
R₁ and R₂ are each independently selected from the group consisting of OH, SH, substituted or unsubstituted C1-6 alkoxy, substituted or unsubstituted C1-6 alkylthio; or R₁ and R₂ taken together with the carbon atom to which they are attached form a substituted or unsubstituted five- to seven-membered ring; and the five- to seven-membered ring is selected from the group consisting of 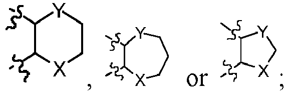 wherein, X and Y are each independently selected from: C(R_{c})₂, O, S, NRₐ, C(=O), C(=S);
in R₁ and R₂, the term "substituted" means that a hydrogen in the group is replaced by one or more (preferably 1-3) substituents selected from the group consisting of halogen, C1-6 alkyl, halogenated C1-6 alkyl, cyano;
R₃ is selected from the group consisting of C(R_{c})₂, C=O, C=S, CR_{c}(OH), CR_{c}(SH);
n is 0, 1, 2, 3 or 4;
R₄ is a divalent group selected from the group consisting of substituted or unsubstituted C2-C6 alkenylene, substituted or unsubstituted C2-C6 alkynylene, NRₐ, substituted or unsubstituted ring G group; wherein, the ring G is selected from the group consisting of a 4- to 18-membered heterocyclic ring, a 5- to 18-membered heteroaromatic ring, a C3-C14 aromatic ring, and a C3-C18 carbocyclic ring;
in R₄, the term "substituted" means that one or more hydrogens in the R₄ group are each independently replaced by R' and/or R"; with the proviso that when R₄ is a ring G group, the ring G can further optionally comprise a oxo and/or a thio;
R₅ is selected from the group consisting of H, nitro, halogen, cyano, substituted or unsubstituted C1-C12 alkyl, substituted or unsubstituted C1-C12 alkoxy, substituted or unsubstituted C1-C12 alkylcarbonyl, -(C(R_{c})₂)ₒ-OCOR_{b}, -(C(R_{c})₂)ₒ-COOR_{b}, -(C(R_{c})₂)ₒ-NRₐ-COOR_{b}, -(C(R_{c})₂)ₒ-NRₐ-OCOR_{b}, N(Rₐ)₂, substituted or unsubstituted ring E group; wherein the ring E group is selected from the group consisting of 4- to 18-membered heterocyclic group, 5- to 18-membered heteroaryl group, C6- C14 aryl, C3-C18 cycloalkyl; in R₅, the term "substituted" means that one or more hydrogens in the R₅ group are replaced by R' and/or R"; with the proviso that when R₅ is a ring E group, the ring E group can further optionally comprise a oxo and/or a thio;
o = 0, 1 or 2;
R' is each independently selected from the group consisting of hydroxyl, thiol, nitro, halogen, cyano, substituted or unsubstituted C1-C10 alkyl, N(Rₐ)₂, substituted or unsubstituted C1-C10 alkoxy, substituted or unsubstituted C1-C10 alkylthio, substituted or unsubstituted C1-C10 alkylcarbonyl, - COOR_{b}, -OCOR_{b}, substituted or unsubstituted 4- to 12-membered heterocyclic group, substituted or unsubstituted 5- to 12-membered heteroaryl, substituted or unsubstituted C6-C14 aryl, substituted or unsubstituted C3-C15 carbocyclic group;
R" is each independently selected from the group consisting of H, hydroxyl, thiol, C1-C6 alkyl, C1-C6 haloalkyl;
Rₐ, R_{b} and R_{c} are each independently selected from the group consisting of H, C1-6 alkyl, C1-C6 haloalkyl;
unless otherwise specified, the term "substituted" means that one or more hydrogens in the group are replaced by a substituent selected from the group consisting of cyano, hydroxy, halogen, C1-6 alkyl, and C1-6 haloalkyl.

2. The compound of claim 1, wherein 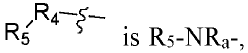 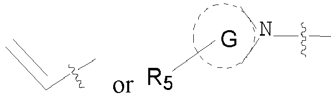 ; wherein, 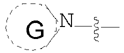 represents ring G, and the ring G is a heterocyclic ring or heteroaromatic ring containing at least one N heteroatom.

3. The compound of claim 1, where 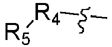 is a group selected from the following group that is unsubstituted or substituted with 1 to 3 R' and/or R": R₅-NRₐ-,

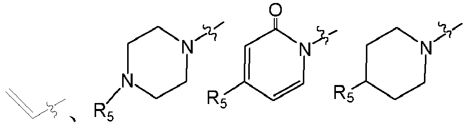

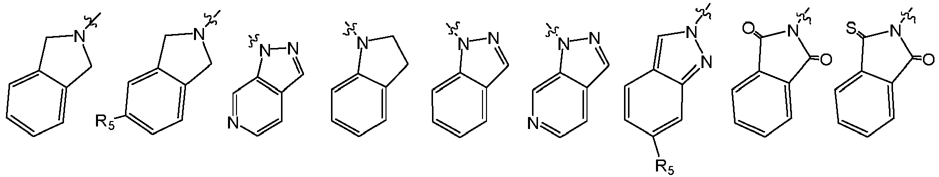

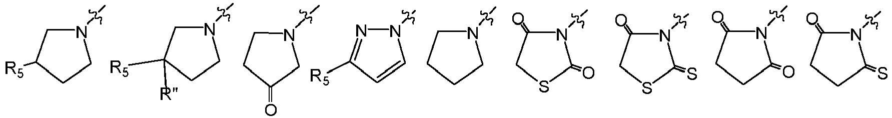

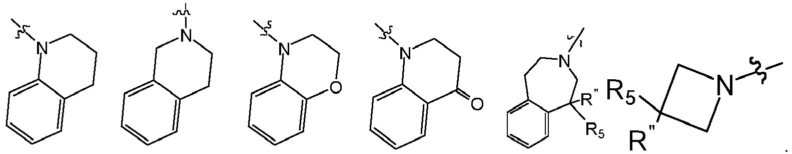

4. The compound of claim 1, wherein the ring E group is selected from the group consisting of:

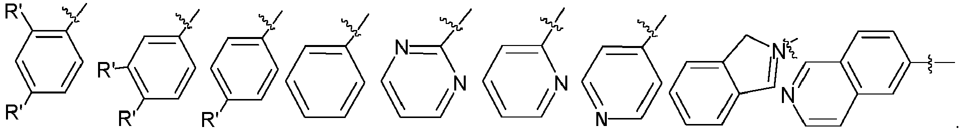

5. The compound of claim 1, wherein the compound is represented by formula IV-A, formula IV-B, formula IV-C, formula IV- or formula IV-D, 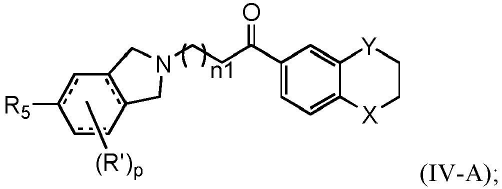 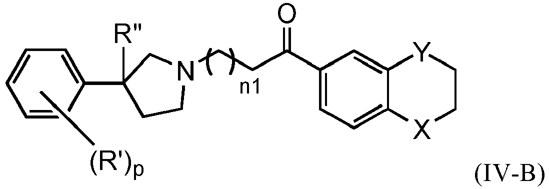 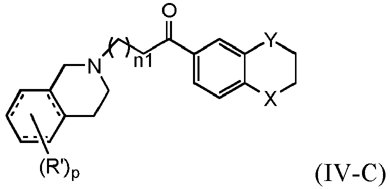 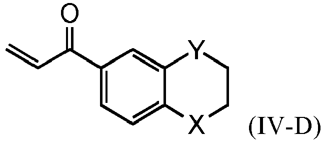 wherein, p=0, 1 or 2; n1=1, 2 or 3; Rs, R', R", Y and X are as defined in claim 1.

6. The compound of claim 1, wherein the compound is selected from the group consisting of:
| | | | | | |
|---|---|---|---|---|---|
| **1** | 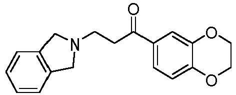 | 1 9 | 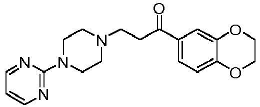 | 3 7 | 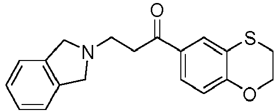 |
| **2** | 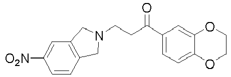 | 2 0 | 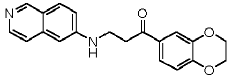 | 3 8 | 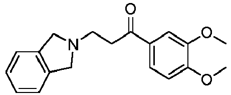 |
| **3** | 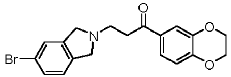 | 2 1 | 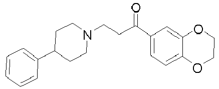 | 3 9 | 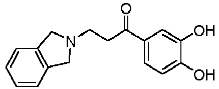 |
| 4 | 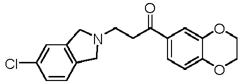 | 2 2 | 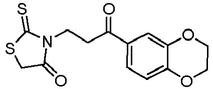 | 4 0 | 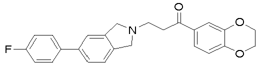 |
| 5 | 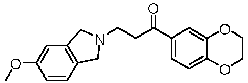 | 2 3 | 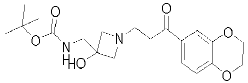 | 4 1 | 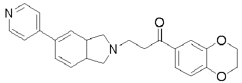 |
| 6 | 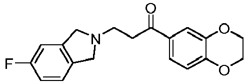 | 2 4 | 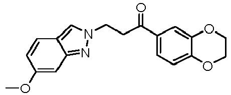 | 4 2 | 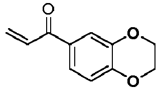 |
| 7 | 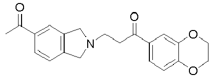 | 2 5 | 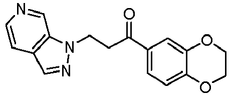 | 4 3 | 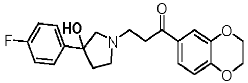 |
| 8 | 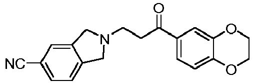 | 2 6 | 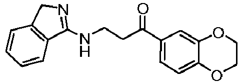 | 4 4 | 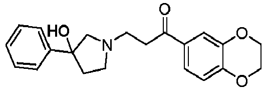 |
| 9 | 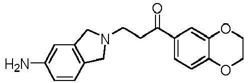 | 2 7 | 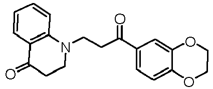 | 4 5 | 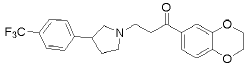 |
| 1 0 | 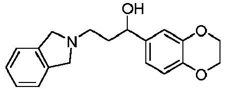 | 2 8 | 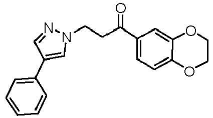 | 4 6 | 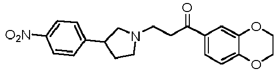 |
| 1 1 | 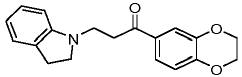 | 2 9 | 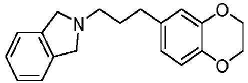 | 4 7 | 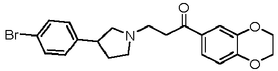 |
| 1 2 | 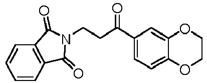 | 3 0 | 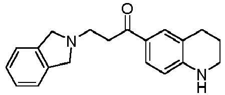 | 4 8 | 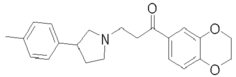 |
| 1 3 | 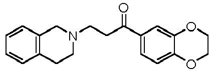 | 3 1 | 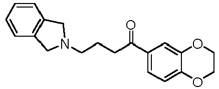 | 4 9 | 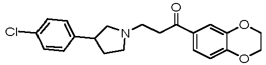 |
| 1 4 | 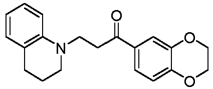 | 3 2 | 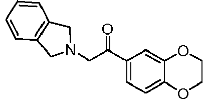 | 5 0 | 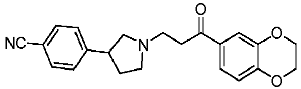 |
| 1 5 | 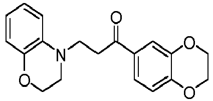 | 3 3 | 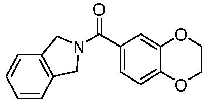 | 5 1 | 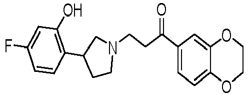 |
| 1 6 | 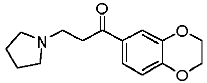 | 3 4 | 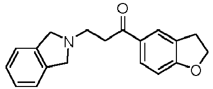 | 5 2 | 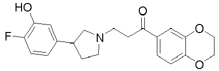 |
| 1 7 | 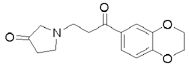 | 3 5 | 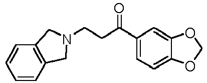 | 5 3 | 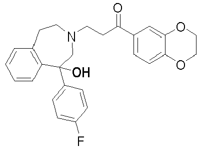 |
| 1 8 | 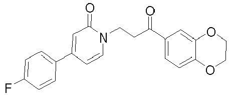 | 3 6 | 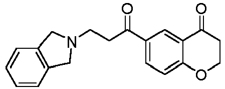 | 5 4 | 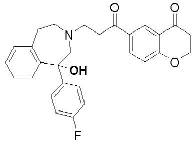 |

7. A method for preparing a compound, wherein the method comprises: 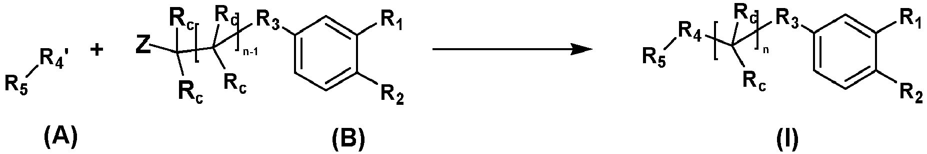 reacting a compound of formula A with a compound of formula B to obtain a compound of formula I;
wherein, Z is a leaving group;
n is 1, 2 or 3;
R₅-R₄' is selected from the group consisting of: 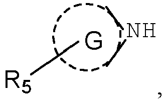 R₅-N(Rₐ)H;
R₁, R₂, R₃, R₅ and R_{c} are are as defined in claim 1.

8. A pharmaceutical composition, comprising (i) the compound of claim 1, and (ii) a pharmaceutically acceptable carrier.

9. Use of the compound of claim 1, or a salt, or an isomer thereof, or a pharmaceutical composition of claim 8 (i) for the preparation of a TDG inhibitor, or (ii) for the preparation of a medicament for treating and/or preventing a disease related to TDG overexpression.

10. The use of claim 9, wherein the disease related to TDG overexpression is a tumor.

11. The use of claim 10, wherein the tumor is selected from the group consisting of lung cancer, colorectal cancer, melanoma, breast cancer, liver cancer, glioma, kidney cancer, pancreatic cancer, ovarian cancer, gastric cancer, neuroblastoma, or a combination thereof.

12. A method for inhibiting TDG activity, the method comprising:
contacting a subject with an effective amount of the compound of claim 1, or a stereoisomer or a tautomer, or a pharmaceutically acceptable salt, or a hydrate, or a crystal form, or a solvate thereof, or the pharmaceutical composition of claim 8, thereby inhibiting TDG activity.
